# EUROPÄISCHE PATENTSCHRIFT

(11) **EP 3 381 347 B1**
(45) Veröffentlichungstag und Bekanntmachung des Hinweises auf die Patenterteilung: **24.06.2020**
(21) Anmeldenummer: 18164491.5
(22) Anmeldetag: 28.03.2018
(51) Int. Cl.: A61B 1/00, A61B 1/002

(54) **BEOBACHTUNGSINSTRUMENT MIT OPTISCHER ANORDNUNG**
OBSERVING INSTRUMENT WITH OPTICAL ASSEMBLY
INSTRUMENT D'OBSERVATION POURVUE DE DISPOSITIF OPTIQUE

(30) Priorität: 29.03.2017 DE 102017106714
(43) Veröffentlichungstag der Anmeldung: 03.10.2018
(73) Patentinhaber: Karl Storz SE & Co. KG Intellectual Property, 78532 Tuttlingen (DE)
(72) Erfinder: Eisenkolb, Peter, 78532 Tuttlingen (DE); Eisenlauer, Johannes, 78532 Tuttlingen (DE); Maichle, Stefanie, 78532 Tuttlingen (DE); Wanner, Florian, 78532 Tuttlingen (DE)
(74) Vertreter: Witte, Weller & Partner Patentanwälte mbB

(56) Entgegenhaltungen:
- WO-A1-98/43529
- WO-A1-99/39623
- DE-A1-102010 050 932
- FR-A1- 2 317 673
- US-A- 5 601 525
- US-A1- 2005 192 479
- US-B2- 6 955 644

## Beschreibung

Die vorliegende Offenbarung betrifft ein Beobachtungsinstrument, insbesondere Endoskop, das mit einem Instrumentenschaft versehen ist, der ein distales Ende und ein proximales Ende aufweist. Ferner umfasst das Beobachtungsinstrument eine optische Anordnung, die sich zumindest teilweise durch den Instrumentenschaft erstreckt. Die optische Anordnung weist zumindest ein optisches Bauelement auf, das in einem Trägerschaft aufgenommen ist.

Beobachtungsinstrumente der vorgenannten Art sind im Stand der Technik bekannt. Beispielhaft handelt es sich um medizinische Beobachtungsinstrumente. Jedoch sind ferner auch Beobachtungsinstrumente für technische Anwendungen denkbar, etwa für die Bauteilkontrolle und für die Diagnosezwecke.

Beobachtungsinstrumente können insbesondere als Endoskope gestaltet sein. Dies kann ferner Beobachtungsinstrumente für spezielle Anwendungszwecke umfassen, bspw. für die Laparoskopie sowie für andere medizinische Gebiete.

Es erfolgt bei Endoskopen regelmäßig eine Unterteilung in starre Endoskope und flexible Endoskope. Jeder Typ hat spezifische Vorteile und Nachteile. Flexible Endoskope weisen regelmäßig eine biegsame Schaftanordnung auf, wobei regelmäßig Lichtleitfasern zur Übertragung des Beleuchtungslichtes und/oder der optischen Abbildung vorgesehen sind.

Die vorliegende Offenbarung bezieht sich in verschiedenen Ausführungsbeispielen insbesondere auf sogenannte starre Endoskope mit einer starren Schaftanordnung. In der Schaftanordnung sind regelmäßig optische Bauelemente vorgesehen, um an einem proximalen Ende der Schaftbaugruppe und/oder des Endoskops eine optische Abbildung eines beobachteten Objektes bereitzustellen. Die Abbildung ist beispielwiese für einen Beobachter an einem Okular darstellbar. Die Abbildung kann jedoch auch in eine Sensorebene eines Bildsensors am proximalen Ende der Schaftbaugruppe und/oder des Endoskops erfolgen.

Aus der DE 10 2011 078 968 A1 ist ein Endoskop mit seitlicher Blickrichtung bekannt, umfassend einen proximalen Griff, einen Endoskopschaft mit einem äußeren Rohr und einem inneren Rohr, zwei im distalen Bereich des Endoskopschafts angeordnete, gegeneinander um eine Längsachse des Endoskopschafts drehbare optische Baugruppen, die durch ein mittels einer Vorspannvorrichtung vorgespanntes, zwischen den optischen Baugruppen angeordnetes Axiallager gegeneinander gelagert sind, wobei eine distal mit dem äußeren Rohr verbundene erste optische Baugruppe seitwärts blickende optische Fenster, Prismen und/oder Linsen umfasst und eine distal mit dem inneren Rohr verbundene zweite optische Baugruppe einen geradeaus blickenden Bildsensor umfasst, der in Richtung der Längsachse des Endoskopschafts ausgerichtet ist.

Das Dokument DE 10 2016 207 275 A1, welches nach dem Prioritätstag der vorliegenden Offenbarung veröffentlicht wurde, zeigt eine ähnliche Gestaltung.

Aus der DE 10 2010 050 932 A1, der DE 199 12 656 A1, der DE 102 53 559 A1 und der DE 12 48 224 A sind weitere Gestaltungen von Schaftanordnungen für Endoskope bekannt.

Aus der US 2005/192479 A1 ist ein starres Endoskop bekannt, mit einem Faserbildleiter, der in einem Führungsrohr verlegt ist, das fest mit dem Endoskop verbunden ist, wobei vor dem distalen Ende des Faserbildleiters im Führungsrohr ein Objektiv und vor dem proximalen Ende des Faserbildleiters außerhalb des Führungsrohres ein Okular angeordnet ist, wobei der Faserbildleiter axial verschiebbar im Führungsrohr gelagert und an seinem proximalen Ende lösbar am Endoskop befestigt ist.

Aus der EP 1 839 563 A1 ist ein Endoskop mit einer Stablinsenanordnung bekannt, die zwischen einer distalen, objektseitigen Optik und einer proximalen, okularseitigen Optik angeordnet ist. Eine solche Anordnung kann auch als Hopkins-Stablinsensystem bezeichnet werden.

Aus der WO 98/43529 A1 ist ein Endoskop mit einem Außenrohr und einem Innenrohr bekannt, wobei im Innenrohr eine optische Anordnung aufgenommen ist, die eine Objektivbaugruppe, eine Okularbaugruppe und eine diesen zwischengeordnete Stablinsenbaugruppe umfasst. Das Innenrohr ist an seinem distalen Ende mit dem Außenrohr fest verbunden. Das Außenrohr ist an seinem proximalen Ende an einem Kopf aufgenommen, der als Gehäuse dient. Zwischen der Okularbaugruppe und der Stablinsenbaugruppe ist eine Feder vorgesehen, die die Stablinsenbaugruppe nach distal vorspannt.

Aus der US 6,955,644 B2 ist ein Endoskop bekannt, das eine Schaftbaugruppe umfasst, die ein Außenrohr, ein Innenrohr und einen inneren Schaft umfasst. Das Innenrohr ist im Außenrohr aufgenommen. In einem Zwischenraum zwischen dem Außenrohr und dem Innenrohr sind Lichtleitfasern angeordnet. Der innere Schaft ist im Innenrohr angeordnet. Der innere Schaft ist nach außen abgedichtet und trägt empfindliche optische Bauelemente. Der innere Schaft ist an seinem distalen Ende mit dem Innenrohr fest verbunden. Im inneren Schaft sind optische Bauelemente aufgenommen, die durch eine Feder nach distal vorgespannt werden, die sich an einem proximalen Ende des inneren Schafts abstützt.

Im Rahmen dieser Offenbarung beschreibt der Begriff "proximal" einen Bereich bzw. einen Abschnitt einer Einheit oder Komponente, der vom zu beobachtenden Objekt abgewandt und einem Beobachter oder Bediener des Beobachtungsinstruments zugewandt ist. Demgemäß beschreibt der Begriff "distal" einen Bereich, der dem zu beobachtenden Objekt zugewandt und vom Beobachter bzw. Benutzer des Beobachtungsinstruments abgewandt ist. Bei einem medizinischen Endoskop, das zum Einführen in Körperöffnungen ausgestaltet ist, wird regelmäßig das distale Ende des Instrumentenschafts in dem Körper eingeführt. Das proximale Ende des Instrumentenschafts bzw. des Beobachtungsinstruments verbleibt hingegen außerhalb des Körpers.

Der Instrumentenschaft eines Beobachtungsinstruments weist regelmäßig eine ausgeprägte Längenerstreckung auf, also ein großes Längen-Durchmesser-Verhältnis. Allgemein ist es gewünscht, den Instrumentenschaft mit möglichst geringem Durchmesser auszuführen. Dies kann Ausgestaltungen umfassen, die - je nach Anwendungsgebiet - etwa einen Schaftdurchmesser des Instrumentenschafts im Bereich von 12 mm bis 10 mm, bis hin zu Durchmessern im Bereich von 8 mm bis 6 mm oder gar im Bereich von 5 mm bis 4 mm umfassen. Auf diese Weise ist es - im Falle einer medizinischen Anwendung - möglich, das Körperinnere eines Patienten zu beobachten, wobei nur geringe Körperöffnungen erforderlich sind, um das Beobachtungsinstrument einzuführen. In gleichem Maße ergeben sich auch bei technischen Anwendungen Vorteile, wenn Instrumentenschäfte verwendet werden, die nur einen kleinen Durchmesser aufweisen.

Die optische Anordnung eines Beobachtungsinstruments umfasst üblicherweise optische Bauelemente, die, je nach ihrer Lage in Bezug auf ein zu beobachtendes Objekt bzw. auf einen Beobachter einer Objektivbaugruppe oder einer Okularbaugruppe zugehörig sind, oder zwischen diesen angeordnet sein können. Andere Gestaltungen sind denkbar. Eine Bauart von Beobachtungsinstrumenten umfasst ferner eine Stablinsenanordnung zwischen einer Objektivbaugruppe und einer Okularbaugruppe einer optischen Anordnung. Die Stablinsenanordnung kann bspw. ein sog. Hopkins-Linsensystem umfassen.

Die Bezeichnung Okularbaugruppe soll nicht einschränkend dahingehend verstanden werden, dass das Bild durch die Okularbaugruppe zwingend für die unmittelbare Beobachtung durch das menschliche Auge bereitgestellt wird. Alternativ oder zusätzlich ist es vorstellbar, die Okularbaugruppe mit einem Bildaufnehmer, etwa mit einem CCD-Chip und/oder einem CMOS-Chip zu koppeln, um eine digitale Abbildung bereitzustellen. Es sind auch Beobachtungsinstrumente bekannt, die für die direkte Beobachtung mit dem menschlichen Auge und für die Verwendung von Bildaufnehmern umrüstbar sind.

Allgemein erlaubt die Verwendung von Stablinsensystemen eine lichtstarke Bildwiedergabe. Dies kann weiter dazu beitragen, den Durchmesser des Instrumentenschafts zu verringern bzw. das Längen-Durchmesser-Verhältnis zu erhöhen.

Ferner sind Beobachtungsinstrumente bekannt, die eine geneigte bzw. seitliche Blickrichtung aufweisen, die zu einer Längsachse des Instrumentenschafts winkelversetzt ist. Hierbei kann es sich um einen fix vorgegebenen seitlichen Blickwinkel handeln. Ferner sind Endoskope bekannt, die veränderliche Blickwinkel aufweisen und zu diesem Zweck bspw. objektivseitig mit Schwenkprismen oder ähnlichen Anordnungen versehen sind.

Zur Wahrung der Abbildungsqualität ist es wichtig, Verschmutzungen der optischen Anordnung zu vermeiden. Dies gilt sowohl für Beobachtungsinstrumente für technische Anwendungen als auch für medizinische Anwendungen. Ferner müssen medizinische Beobachtungsinstrumente regelmäßig intensiv gereinigt bzw. sterilisiert werden, um eine Anwendung am menschlichen oder tierischen Körper zu ermöglichen. Üblicherweise werden medizinische Beobachtungsinstrumente, zumindest Baugruppen hiervon, regelmäßig autoklaviert. Auf diese Weise erfolgt eine Sterilisation.

Die vorgenannten Anforderungen bedingen einerseits eine hinreichende Abdichtung zumindest der optischen Anordnung, die jedoch üblicherweise auch weitere Bauteile des Beobachtungsinstruments betrifft. Ferner müssen bei der Gestaltung von Beobachtungsinstrumenten Randbedingungen und Parameter der Sterilisation beachtet werden. Dies betrifft einerseits eine erhöhte Temperatur und ferner einen erhöhten Druck bzw. einen bestimmten Feuchtegehalt, insbesondere bei der sog. DampfdruckSterilisation.

Ferner ist bei der Gestaltung von Beobachtungsinstrumenten stets die Bauraumminimierung, insbesondere die Durchmesserminimierung, zu beachten. Eine weitere Herausforderung für die Gestaltung und Fertigung von Beobachtungsinstrumenten ist eine hochgenaue Montage und Justage der beteiligten optischen Bauelemente. Auf diese Weise kann die gewünschte Abbildungsqualität gewährleistet werden.

Bei der Gestaltung von als Endoskopen ausgeführten optischen Beobachtungsinstrumenten hat sich eine Ausführung mit mehreren ineinander angeordneten Rohren durchgesetzt. Dies kann bspw. ein Außenrohr und ein darin aufgenommenes Innenrohr umfassen, vgl. die obige WO 98/43529 A1. Eine weitere Möglichkeit ist die sog. Drei-Rohr-Technik, vgl. auch die obige US 6,955,644 B2, die ein Endoskop beschreibt, das eine Schaftbaugruppe mit drei ineinander angeordneten Rohren umfasst.

Diese Gestaltung erlaubt eine sichere Abdichtung der optischen Anordnung und weiterer Komponenten des Endoskops. Es hat sich jedoch gezeigt, dass die Montage derartiger Schaftbaugruppen mit hohem Aufwand verbunden ist. Dies betrifft insbesondere die erforderliche Verbindungstechnik zur Fixierung der einzelnen Elemente. Etablierte Verbindungstechniken bei Beobachtungsinstrumenten umfassen bspw. formschlüssige Verbindungen, stoffschlüssige Verbindungen, kraftschlüssige Verbindungen, reibschlüssige Verbindungen, usw.

Stoffschlüssige Verbindungen können etwa Schweißen, Löten und Kleben umfassen. Kraftschlüssige bzw. reibschlüssige Verbindungen umfassen bspw. Schraubverbindungen. Formschlüssige Verbindungen umfassen bspw. Sicherungselemente, wie etwa Sicherungsringe, Schnappverschlüsse, und dergleichen. Es ist mit gewissen Schwierigkeiten verbunden, sowohl am distalen Ende als auch am proximalen Ende der Schaftbaugruppe die beteiligten Schäfte bzw. Rohre und ggf. darin aufgenommene Komponenten sicher und mit der erforderlichen Genauigkeit anzuordnen und miteinander zu fügen.

Ferner hat sich gezeigt, dass Beobachtungsinstrumente anfällig für Erschütterungen sind. Derartige mechanische Belastungen können zu einer Beschädigung des Instruments führen. Gleichermaßen können sich durch Sterilisationsvorgänge, Reinigungsvorgänge und Ähnliches Bauteilbelastungen ergeben, die ebenso zu Beschädigungen führen können.

Vor diesem Hintergrund liegt der Offenbarung die Aufgabe zugrunde, ein Beobachtungsinstrument anzugeben, bei dem die optische Anordnung einfach montierbar und mit hoher Positioniergenauigkeit im Instrumentenschaft aufgenommen ist. Das Beobachtungsinstrument soll möglichst robust und unempfindlich gestaltet sein. Das Beobachtungsinstrument soll möglichst einfach zu reinigen und zu sterilisieren sein. Ferner soll die optische Anordnung mechanischen Belastungen und Stößen standhalten können, ohne dass sich dies nachteilig auf die Funktion und die mechanische Integrität des Beobachtungsinstruments auswirkt. Ferner soll möglichst ein Montagekonzept für die optische Anordnung eines Beobachtungsinstruments angegeben werden, das insgesamt eine Gestaltung des Instrumentenschafts mit möglichst kleinem Durchmesser ermöglicht. Die Montage soll einfach und wiederholgenau erfolgen können. Darüber hinaus soll ein Beobachtungsinstrument gemäß der vorliegenden Offenbarung Verbesserungen im Hinblick auf zumindest einen der vorgenannten Nachteile ermöglichen. Die optische Anordnung soll möglichst hochgenau positioniert werden können, insbesondere im Hinblick auf eine Anordnung einer Objektivbaugruppe der optischen Anordnung in Bezug auf ein distales Ende des Instrumentenschafts. Gleichwohl soll eine möglichst zuverlässige und haltbare Anordnung und Anbindung der optischen Bauelemente gewährleistet werden.

Diese Aufgabe wird erfindungsgemäß durch ein Beobachtungsinstrument, insbesondere ein Endoskop, gelöst, mit einem Instrumentenschaft mit einem distalen Ende und einem proximalen Ende, und mit einer optischen Anordnung, die sich zumindest abschnittsweise durch den Instrumentenschaft erstreckt, wobei die optische Anordnung zumindest ein optisches Bauelement aufweist, das in einem Trägerschaft aufgenommen ist, wobei der Trägerschaft im Instrumentenschaft zumindest abschnittsweise axial verschieblich aufgenommen und nach distal vorgespannt ist, wobei der Trägerschaft durch ein proximal angeordnetes Vorspannelement, das auf den Trägerschaft einwirkt, nach distal vorgespannt ist, und wobei die optische Anordnung im Trägerschaft durch ein weiteres Vorspannelement zumindest abschnittsweise relativ zum Trägerschaft nach distal vorgespannt ist.

Die Aufgabe der Erfindung wird auf diese Weise vollkommen gelöst.

Erfindungsgemäß wird nämlich der Trägerschaft kraftschlüssig unter Vorspannung im Instrumentenschaft aufgenommen, wobei eine Kraftkomponente den Trägerschaft nach distal vorspannt. Auf diese Weise ist der Trägerschaft mitsamt der zumindest teilweise darin aufgenommenen optischen Anordnung kraftschlüssig im Instrumentenschaft gehalten. Dies hat den Vorteil, dass insbesondere ein distales Ende des Trägerschafts hochgenau in Bezug auf das distale Ende des Instrumentenschafts positioniert werden kann. Der Trägerschaft ist kraftschlüssig im Instrumentenschaft gehalten. Die kraftschlüssige Aufnahme unter Vorspannung erlaubt eine zumindest teilweise elastische, nachgiebige und fehlerkompensierende Lagerung oder Aufnahme optischer Bauelemente der optischen Anordnung. Auf diese Weise kann sich eine Kompensation von Temperaturschwankungen und damit verbundenen Dehnungen ergeben. Ferner kann sich eine gewisse Robustheit gegenüber Stößen und anderen mechanischen Belastungen ergeben. Auch die Empfindlichkeit gegenüber Vibrationen, Schwingungen und ähnlichen Einflussfaktoren sinkt deutlich. Insgesamt kann sich auf diese Weise auch eine Montagevereinfachung ergeben, wenn der Trägerschaft, ausgehend von seinem proximalen Ende, in Richtung auf das distale Ende des Instrumentenschafts vorgespannt wird. Mit anderen Worten ist es beim distalen Ende des Trägerschafts nicht unbedingt erforderlich, Verbindungselemente vorzusehen, die dort einen Eingriff während der Montage erforderlich macht.

Der Trägerschaft ist einseitig vorgespannt. Dies kann eine kraftschlüssige Lagerung umfassen, also eine Positionierung unter Vorspannung. Der Trägerschaft ist demgemäß nicht an seinen beiden Enden (distales Ende und proximales Ende) fest eingespannt. Stattdessen ergibt sich aufgrund der Vorspannung am distalen Ende eine genaue Positionierung. Die resultierende Positionierung (relativ zum Instrumentenschaft bzw. zu einem Gehäuse des Beobachtungsinstruments) am proximalen Ende erfolgt unter Vorspannung und kann folglich Ausgleichsbewegungen umfassen. Die proximale

Positionierung ist somit nicht fix, sondern zumindest in Grenzen veränderlich. Insgesamt ist der Trägerschaft zumindest in engen Grenzen, die durch die Elastizität der Vorspannung (also etwa einer Feder) definiert sind, relativ zum Gehäuse axial verfahrbar. Der Trägerschaft ist zumindest geringfügig axial beweglich im Instrumentenschaft angeordnet.

Die gefederte, geringfügig bewegliche Lagerung zumindest einiger optischer Bauelemente kann sich insbesondere dann ergeben, wenn diese optischen Bauelemente - zusätzlich zur Aufnahme des Trägerschafts im Instrumentenschaft unter Vorspannung - im Trägerschaft unter Vorspannung aufgenommen sind. Mit anderen Worten kann der Trägerschaft als Hülle oder Mantel für zumindest einige der optischen Bauelemente dienen. Zumindest einige der optischen Bauelemente können durch ein Vorspannelement, das sich mittelbar oder unmittelbar am Trägerschaft abstützt, nach distal vorgespannt werden. Zusätzlich kann jedoch der Trägerschaft selbst über ein Vorspannelement, das sich gehäuseseitig abstützt, gegenüber dem Instrumentenschaft nach distal vorgespannt werden. Der Instrumentenschaft selbst ist regelmäßig gehäusefest aufgenommen, also hinreichend fest mit einem Kopf oder Gehäuse des Beobachtungsinstruments verbunden.

Der Trägerschaft ist in beispielhaften Ausführungsformen nicht nur als kurze Trägerhülse gestaltet, sondern als Schaft mit ausgeprägter Längserstreckung, der sich zwischen einem proximalen Ende und einem distalen Ende der Schaftbaugruppe erstreckt. In beispielhaften Ausgestaltungen ist die Längserstreckung des Trägerschafts nahezu gleich groß wie oder sogar größer als die Längserstreckung des (äußeren) Instrumentenschafts. Mit anderen Worten erstreckt sich bei diesen Ausgestaltungen der Trägerschaft regelmäßig in das Kopfstück bzw. das Gehäuse am proximalen Ende des Endoskops hinein.

Gemäß einer weiteren beispielhaften Ausgestaltung des Beobachtungsinstruments ist der Trägerschaft im Instrumentenschaft axial gegen ein distales Abschlusselement vorgespannt, das lagefest mit dem Instrumentenschaft gekoppelt ist. Diese Abstützung kann mittelbar oder unmittelbar erfolgen. Beispielsweise kann der Trägerschaft, ggf. unter Einbeziehung eines Abstandshalters, das distale Abschlusselement mittelbar oder unmittelbar kontaktieren. Das distale Abschlusselement kann mittelbar oder unmittelbar mit dem Trägerschaft gefügt sein.

Gemäß einer beispielhaften Ausgestaltung weist das Beobachtungsinstrument ferner einen Abstandshalter auf, der zwischen dem Trägerschaft und einem distalen Abschlusselement des Beobachtungsinstruments angeordnet ist, das mit dem Instrumentenschaft gekoppelt ist. Auf diese Weise wird eine genaue Anordnung einer Objektivbaugruppe in Bezug auf das distale Ende des Beobachtungsinstruments vereinfacht. Somit kann sich die Abbildungsqualität insgesamt erhöhen.

Beispielsweise kann es sich bei dem distalen Abschlusselement um ein Fenster oder eine Scheibe handeln. Das distale Abschlusselement ist regelmäßig mittelbar oder unmittelbar mit dem distalen Ende des Instrumentenschafts gekoppelt. Auf diese Weise kann das distale Abschlusselement schlussendlich einen Anschlag für den Trägerschaft bereitstellen, der sich über den Abstandshalter am Abschlusselement abstützt.

Durch den Abstandshalter kann in einfacher Weise ein Abstand zwischen einen am weitesten distal angeordneten Bauelement, das mittelbar oder unmittelbar am Trägerschaft aufgenommen ist, und dem Abschlusselement hochgenau eingestellt werden. Diese Gestaltung schließt nicht aus, dass es sich bei dem Abschlusselement selbst um ein optisch wirksames Bauelement handelt.

Ferner umfasst die obige Ausgestaltung sowohl Beobachtungsinstrumente mit gerader Blickrichtung als auch Beobachtungsinstrumente mit seitlicher Blickrichtung, die gegenüber einer Längsachse des Instrumentenschafts geneigt ist.

Gemäß einer weiteren beispielhaften Ausgestaltung des Beobachtungsinstruments definiert der Abstandshalter einen Abstand zwischen einer Objektivbaugruppe der optischen Anordnung und dem distalen Abschlusselement. Mit anderen Worten drückt der Trägerschaft die Objektivbaugruppe gegen den Abstandshalter und somit, unter Wahrung eines definierten Abstands, gegen das distale Ende des Instrumentenschafts. Gemäß einer beispielhaften Ausgestaltung ist die Objektivbaugruppe zumindest teilweise in einer Trägerhülse der Objektivbaugruppe aufgenommen, die durch den Trägerschaft kontaktiert wird. Es ist grundsätzlich auch vorstellbar, die Objektivbaugruppe zumindest teilweise direkt im Trägerschaft aufzunehmen. Die Objektivbaugruppe kann grundsätzlich mit der Trägerhülse oder dem Trägerschaft verklebt sein.

Gemäß einer weiteren beispielhaften Ausgestaltung des Beobachtungsinstruments erstreckt sich der Trägerschaft ausgehend vom distalen Ende des Instrumentenschafts hin zum proximalen Ende des Instrumentenschafts und vorzugsweise in ein Gehäuse eines Kopfstücks des Beobachtungsinstruments. Es handelt sich demgemäß bei dem Trägerschaft nicht nur um eine vergleichsweise kurze Hülse.

Der Trägerschaft ist durch ein proximal angeordnetes Vorspannelement, das auf den Trägerschaft einwirkt, nach distal vorgespannt. Das Vorspannelement ist vorzugsweise am oder beim proximalen Ende des Trägerschafts angeordnet.

Gemäß einer weiteren beispielhaften Ausgestaltung des Beobachtungsinstruments ist das Vorspannelement zwischen einer proximalen Druckfläche und einer distalen Mitnahmefläche angeordnet, die mit dem Trägerschaft gekoppelt ist. Das Vorspannelement stützt sich an seinem proximalen Ende an der Druckfläche ab. An seinem distalen Ende wirkt das Vorspannelement auf die Mitnahmefläche, die am Trägerschaft aufgenommen bzw. mit dem Trägerschaft verbunden ist. Das Vorspannelement drängt die proximale Druckfläche und die distale Mitnahmefläche auseinander. Die Mitnahmefläche ist bspw. an einer Aufnahmehülse ausgebildet, die mit dem Trägerschaft gekoppelt ist.

Gemäß einer weiteren beispielhaften Ausgestaltung des Beobachtungsinstruments umgibt das Vorspannelement einen Beobachtungsstrahlengang des Beobachtungsinstruments. Mit anderen Worten verläuft der Beobachtungsstrahlengang bzw. der Beobachtungspfad durch das Vorspannelement hindurch. Dies kann eine konzentrische Ausrichtung zwischen dem Vorspannelement und dem Trägerschaft umfassen. Mit anderen Worten kann sich zumindest ein optisches Bauelement zumindest abschnittsweise durch das Vorspannelement erstrecken, wobei das Vorspannelement und das Bauelement axial zueinander ausgerichtet sind.

Gemäß einer weiteren beispielhaften Ausgestaltung des Beobachtungsinstruments ist das Vorspannelement als Druckfeder gestaltet. Insbesondere kann das Vorspannelement als Schraubenfeder ausgeführt sein. Es versteht sich, dass auch fluidische Vorspannelemente, also Gasfedern oder dergleichen, vorstellbar sind. Ferner kann das Vorspannelement grundsätzlich aus Metall, alternativ jedoch auch aus Kunststoffwerkstoffen bestehen.

Die optische Anordnung im Trägerschaft ist zumindest abschnittsweise relativ zum Trägerschaft nach distal vorgespannt. Auf diese Weise kann sich insgesamt eine gefederte, kraftschlüssig gesicherte Aufnahme der optischen Anordnung ergeben. Die Fähigkeit für Ausgleichsbewegung in Reaktion auf Belastungen, Druck- oder Temperaturschwankungen ist gegeben. Somit ist eine hohe Robustheit gewährleistet. Mit anderen Worten kann der Trägerschaft selbst nach distal vorgespannt werden, wobei sich ein Anschlag an einem distalen Ende des Instrumentenschafts bzw. des Beobachtungsinstruments ergibt. Ferner können zumindest einige optische Bauelemente im Trägerschaft nach distal vorgespannt werden, wobei im Trägerschaft selbst - oder damit gekoppelt - ein Anschlag für die nach distal vorgespannten optischen Bauelemente vorgesehen ist. Der Anschlag im oder beim Trägerschaft kann etwa durch die Objektivbaugruppe bereitgestellt werden, die gemäß zumindest beispielhaften Ausführungsformen am distalen Ende des Trägerschafts aufgenommen ist. Dies kann eine stoffschlüssige Fassung der Objektivbaugruppe am Trägerschaft umfassen, etwa mittels Kleben, Löten oder Schweißen.

Gemäß einer weiteren beispielhaften Ausgestaltung des Beobachtungsinstruments ist die optische Anordnung zumindest teilweise im Trägerschaft axial verschieblich aufgenommen, wobei die optische Anordnung zumindest teilweise das weitere Vorspannelement, das als inneres Vorspannelement gestaltet ist, nach distal vorgespannt wird.

Auf diese Weise kann das Beobachtungsinstrument ein äußeres Vorspannelement umfassen, das mit dem Trägerschaft gekoppelt ist, um diesen nach distal vorzuspannen. Ferner kann die optische Anordnung ein inneres Vorspannelement umfassen, das mit im Trägerschaft aufgenommenen optischen Bauelementen gekoppelt ist, um diese nach distal vorzuspannen. Die Vorspannelemente können konzentrisch zueinander angeordnet sein.

Gemäß einer weiteren beispielhaften Ausgestaltung des Beobachtungsinstruments ist das innere Vorspannelement mit zumindest einem optischen Bauelement, insbesondere einer Stablinse, gekoppelt, das im Trägerschaft angeordnet ist. Das innere Vorspannelement spannt das zumindest eine optische Bauelement relativ zum Trägerschaft nach distal vor. Demgemäß kann die optische Anordnung eine Stablinsenanordnung oder Stablinsenbaugruppe umfassen, die sich proximal an die (distal angeordnete) Objektivbaugruppe anschließt. Die Stablinsenanordnung kann als Hopkins-Linsenanordnung ausgeführt sein. Die Stablinsenanordnung umfasst mehrere Stablinsen, die ggf. durch weitere Bauelemente (etwa Korrekturlinsen) und/oder Abstandshalter (Abstandsringe, Spacer) voneinander beabstandet sind. Vorzugsweise ist die Stablinsenbaugruppe vollständig oder nahezu vollständig im Trägerschaft aufgenommen. Das innere Vorspannelement kann die Stablinsenanordnung in Richtung auf einen distalen Anschlag, insbesondere in Richtung auf die Objektivbaugruppe, vorspannen. Insgesamt ergibt sich somit im Trägerschaft eine optische Anordnung, die robust gegenüber Erschütterungen, Schwingungen und sonstigen mechanischen Belastungen ist.

Gemäß einer weiteren beispielhaften Ausgestaltung des Beobachtungsinstruments umfasst die optische Anordnung ferner eine Okularbaugruppe, die proximalseitig des Trägerschafts angeordnet ist. Vorzugsweise stützt sich das innere Vorspannelement an seinem proximalen Ende mittelbar oder unmittelbar an der Okularbaugruppe ab. Somit kann das innere Vorspannelement zwischen der Okularbaugruppe und der Stablinsenbaugruppe angeordnet sein.

Dies kann Gestaltungen umfassen, bei denen das innere Vorspannelement die Okularbaugruppe nach proximal und die Stablinsenbaugruppe nach distal vorspannt. Auf diese Weise kann die Okularbaugruppe in der axialen Richtung nachgiebig gelagert sein, insbesondere im Hinblick auf die axiale Positionierung der Okularbaugruppe im Hinblick auf ein Gehäuse und/oder ein distales Ende des Instrumentenschafts. Dies kann eine "schwimmende" Positionierung der Okularbaugruppe umfassen.

Eine beispielhafte Ausgestaltung umfasst eine fest/starre Kopplung der Okularbaugruppe mit dem Trägerschaft. Somit wird die Okularbaugruppe (etwa über einen Trägertubus) gemeinsam mit dem Trägerschaft durch das äußere Vorspannelement nach distal vorgespannt. Dies kann axiale Relativbewegungen, insbesondere axiale Ausgleichsbewegungen, relativ zum Trägerschaft umfassen. Dies kann Gestaltungen umfassen, bei denen die Axiallage der Okularbaugruppe in Bezug auf den Trägerschaft einstellbar ist, etwa im Wege einer Justierung während der Montage des Beobachtungsinstruments.

Eine weitere beispielhafte Ausgestaltung umfasst eine fest/starre Kopplung der Okularbaugruppe mit dem Instrumentenschaft und oder dem Gehäuse des Beobachtungsinstruments. Somit wird der Trägerschaft durch das äußere Vorspannelement gegenüber dem Instrumentenschaft und gegenüber der Okularbaugruppe nach distal vorgespannt. Dies kann Gestaltungen umfassen, bei denen die Axiallage der Okularbaugruppe in Bezug auf den Instrumentenschaft und oder das Gehäuse einstellbar ist, etwa im Wege einer Justierung während der Montage des Beobachtungsinstruments

Ferner ist gemäß einer weiteren beispielhaften Ausgestaltung des Beobachtungsinstruments ein Drehlagensicherungselement vorgesehen, das eine Drehlage des unter Vorspannung aufgenommenen Trägerschafts in Bezug auf den Instrumentenschaft und/oder das Gehäuse des Beobachtungsinstruments definiert.

Gemäß weiteren beispielhaften Ausgestaltungen ist die Okularbaugruppe zumindest abschnittsweise in einem Gehäuse bzw. Kopf des Beobachtungsinstruments aufgenommen. Demgemäß ist die Okularbaugruppe nicht oder nicht wesentlich im Trägerschaft und/oder im Instrumentenschaft aufgenommen. Wenn die Okularbaugruppe im Gehäuse aufgenommen ist, kann die Okularbaugruppe grundsätzlich einen Durchmesser aufweisen, der größer als ein möglicher Durchmesser ist, der durch den Trägerschaft bereitgestellt wird. Beispielsweise kann das Gehäuse des Beobachtungsinstruments eine axiale Relativlage der Okularbaugruppe definieren. Ferner kann das Gehäuse des Beobachtungsinstruments mittelbar oder unmittelbar einen proximalen Anschlag für die Okularbaugruppe bereitstellen.

Gemäß einer weiteren beispielhaften Ausgestaltung des Beobachtungsinstruments ist zwischen dem Instrumentenschaft und dem Trägerschaft ein Zwischenschaft angeordnet, der den Trägerschaft umgibt. Auf diese Weise kann sich insgesamt eine Drei-Rohr-Anordnung der Schaftbaugruppe des Beobachtungsinstruments ergeben.

Bei dieser Ausgestaltung bildet der Instrumentenschaft einen Außenschaft des Beobachtungsinstruments, der für eine gewisse mechanische Stabilität sorgt. Der Zwischenschaft kann auch als Innenschaft bezeichnet werden, vgl. die US 6,955,644 B2. Zwischen dem Instrumentenschaft und dem Zwischenschaft ist üblicherweise ein Zwischenraum ausgebildet, der etwa zur Aufnahme von Lichtfasern dient. Der Instrumentenschaft und der Zwischenschaft können grundsätzlich konzentrisch zueinander angeordnet sein. Es sind jedoch auch Ausgestaltungen vorstellbar, bei denen der Instrumentenschaft und der Zwischenschaft parallel zueinander angeordnet sind, wobei jedoch Längsachsen des Instrumentenschafts und des Zwischenschafts voneinander beabstandet sind.

Der Zwischenschaft kann eine hermetische Abdichtung der optischen Anordnung bewerkstelligen. Der Zwischenschaft dient zur Aufnahme des Trägerschafts und der darin oder daran aufgenommenen optischen Bauelemente. Demgemäß kann der Trägerschaft als Führung für zumindest Teile der optischen Anordnung dienen. Der Trägerschaft erlaubt eine hochgenaue konzentrische Anordnung der optischen Bauelemente. Der Trägerschaft und der Zwischenschaft können konzentrisch zueinander ausgerichtet sein.

Gemäß beispielhaften Ausgestaltungen ist das distale Abschlusselement am oder im Zwischenschaft aufgenommen. Beim distalen Ende des Beobachtungsinstruments können der Instrumentenschaft und der Zwischenschaft miteinander gekoppelt sein, etwa durch eine stoffschlüssige Verbindung. Gemäß dieser Ausgestaltung ist der Zwischenschaft fest im Instrumentenschaft aufgenommen. Der Trägerschaft wird durch das Vorspannelement in Richtung auf das distale Ende des Zwischenschafts bzw. des Instrumentenschafts vorgespannt und hochgenau positioniert.

Gemäß einer weiteren beispielhaften Ausgestaltung des Beobachtungsinstruments ist der Zwischenschaft beim distalen Ende fest mit dem Instrumentenschaft verbunden. Der Zwischenschaft ist bspw. mit dem Instrumentenschaft verklebt. Es ist auch vorstellbar, ein ringartiges Koppelelement zwischen dem Instrumentenschaft und dem Zwischenschaft anzuordnen. Ferner sind Ausgestaltungen vorstellbar, bei denen das distale Abschlusselement sowohl mit dem Instrumentenschaft als auch mit dem Zwischenschaft fest verbunden ist. Die vorstehend beschriebenen Ausgestaltungen beinhalten, dass keine axiale Relativbewegung zwischen dem Instrumentenschaft und dem Zwischenschaft, zumindest beim distalen Ende, gewünscht ist.

Gemäß einer weiteren beispielhaften Ausgestaltung des Beobachtungsinstruments schließt der Zwischenschaft den Trägerschaft und die darin aufgenommene optische Anordnung nach außen ab. Mit anderen Worten dient der Zwischenschaft als Hülle bzw. Abdichtung für die optische Anordnung. Dies betrifft insbesondere eine radiale und eine distale Abdichtung. Eine proximale Abdichtung kann durch das Gehäuse bewerkstelligt werden.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1: eine perspektivische rückwärtige Ansicht eines als Endoskop gestalteten Beobachtungsinstruments, mit gebrochener Darstellung eines Instrumentenschafts;
- Fig. 2: eine schematische, vereinfachte Schnittansicht eines distalen Bereichs eines Instrumentenschafts;
- Fig. 3: eine Schnittansicht eines proximalen Bereichs eines Trägerschafts, in dem eine Stablinsenbaugruppe angeordnet ist, wobei sich eine Okularbaugruppe an die Stablinsenbaugruppe anschließt;
- Fig. 4: eine Schnittansicht eines Gehäuses oder Kopfstücks eines als Endoskop ausgeführten Beobachtungsinstruments, in dem ein Instrumentenschaft und ein Zwischenschaft angeordnet sind;
- Fig. 5: eine Schnittansicht eines proximalen Abschnitts eines Beobachtungsinstruments, umfassend die Anordnung gemäß Fig. 3 und die Anordnung gemäß Fig. 4 in einem gefügten Zustand;
- Fig. 6: eine Schnittansicht eines distalen Bereichs eines Trägerschafts mit einer daran aufgenommenen Objektivbaugruppe;
- Fig. 7: eine Schnittansicht eines distalen Bereichs eines Instrumentenschafts, in dem eine Objektivbaugruppe angeordnet ist, die an einem Trägerschaft und, über den Trägerschaft, in einem Zwischenschaft aufgenommen ist;
- Fig. 8: eine weitere Schnittansicht eines distalen Bereichs eines Instrumentenschafts, in dem eine Objektivbaugruppe angeordnet ist, die an einem Trägerschaft aufgenommen ist, der in einem Zwischenschaft angeordnet ist; und
- Fig. 9: eine geschnittene Teilansicht eines Kopfbereichs eines Beobachtungsinstruments, in dem eine Okularbaugruppe angeordnet ist, die sich proximal an eine Stablinsenbaugruppe anschließt.

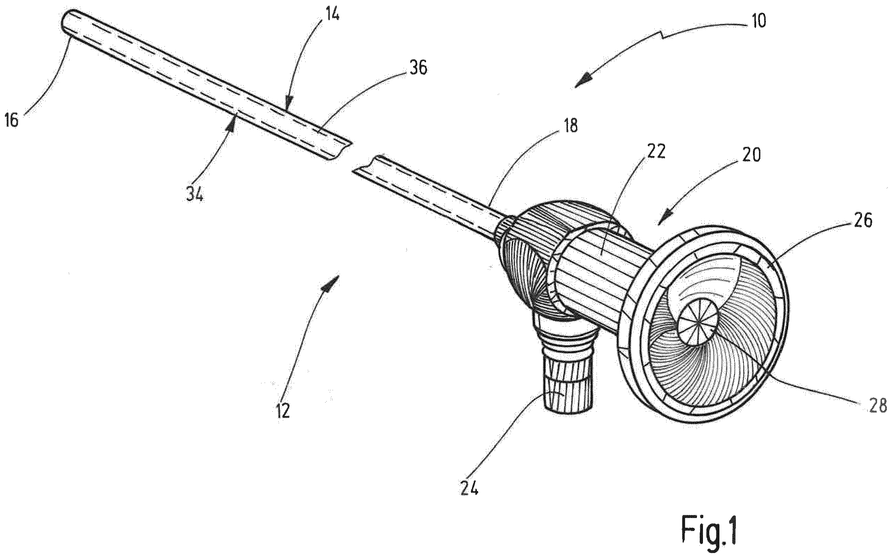

Fig. 1 zeigt eine perspektivische rückwärtige Ansicht einer beispielhaften Ausführungsform eines Beobachtungsinstruments 10, das als Endoskop 12 gestaltet ist. Anwendungsgebiete für das Beobachtungsinstrument 10 können medizinische Zwecke, ferner jedoch auch technische Zwecke betreffen.

Das Instrument 10 umfasst einen Instrumentenschaft 14, der in Fig. 1 gebrochen dargestellt ist. Der Instrumentenschaft 14 weist ein ausgeprägtes Längen-Durchmesser-Verhältnis auf. Bei der Nutzung für medizinische Zwecke kann der Instrumentenschaft 14 in Körperöffnungen eingeführt werden, um Beobachtungen zu ermöglichen.

Der Instrumentenschaft 14 umfasst ein distales Ende 16 und ein proximales Ende 18. Das distale Ende 16 des Instrumentenschafts 14 definiert ein distales Ende des Instruments 10 an das proximale Ende 18 des Instrumentenschafts 14 schließt sich ein Kopfstück 20 an, das auch als Gehäuse 22 bezeichnet werden kann. Beispielhaft ist am Gehäuse 22 ferner ein Lichtanschluss 24 ausgebildet, über den Lichtleiter oder dergleichen angekoppelt werden können. Am Gehäuse 22 ist ferner eine Okularmuschel 26 aufgenommen, die ein Okularfenster 28 umgibt. Die Okularmuschel 26 ist an einem proximalen Ende des Gehäuses 22 aufgenommen, das einem distalen Ende des Gehäuses 22 gegenüberliegt, von dem sich der Instrumentenschaft 14 in Richtung auf das distale Ende 16 des Instruments 10 erstreckt. Es versteht sich, dass das Instrument 10 alternativ oder zusätzlich zur Okularmuschel 26 mit dem Okularfenster 28 einen Bildaufnehmer aufweisen kann. Ein derartiges Instrument 10 kann auch als okularloses Instrument bezeichnet werden. Dies schließt jedoch nicht aus, dass beim Instrument 10 eine sog. Okularbaugruppe vorgesehen ist.

Das Instrument 10 umfasst eine optische Anordnung 34, die in Fig. 1 durch eine gestrichelte Darstellung angedeutet ist. Die optische Anordnung 34 erstreckt sich zumindest abschnittsweise durch den Instrumentenschaft 14. Die optische Anordnung 34 erstreckt sich vorzugsweise ausgehend von einem distalen Ende 16 des Instrumentenschafts 14 in das Gehäuse 22 hinein. Je nach Betrachtungsweise kann auch das Okularfenster 28 einen Bestandteil der optischen Anordnung 34 bilden. Die optische Anordnung 34 weist eine Mehrzahl optischer Bauelemente auf. Hierbei kann es sich beispielhaft um eine Objektivbaugruppe handeln, die beim distalen Ende 16 des Instrumentenschafts 14 angeordnet ist. Ferner kann die optische Anordnung 34 eine Okularbaugruppe umfassen, die bspw. im Gehäuse 22 aufgenommen ist. Zwischen der Objektivbaugruppe und der Okularbaugruppe kann eine Stablinsenanordnung angeordnet sein, die auch als Hopkins-Linsenanordnung ausgeführt sein kann. Die optische Anordnung 34 definiert einen Beobachtungsstrahlengang 36 durch das Instrument 10.

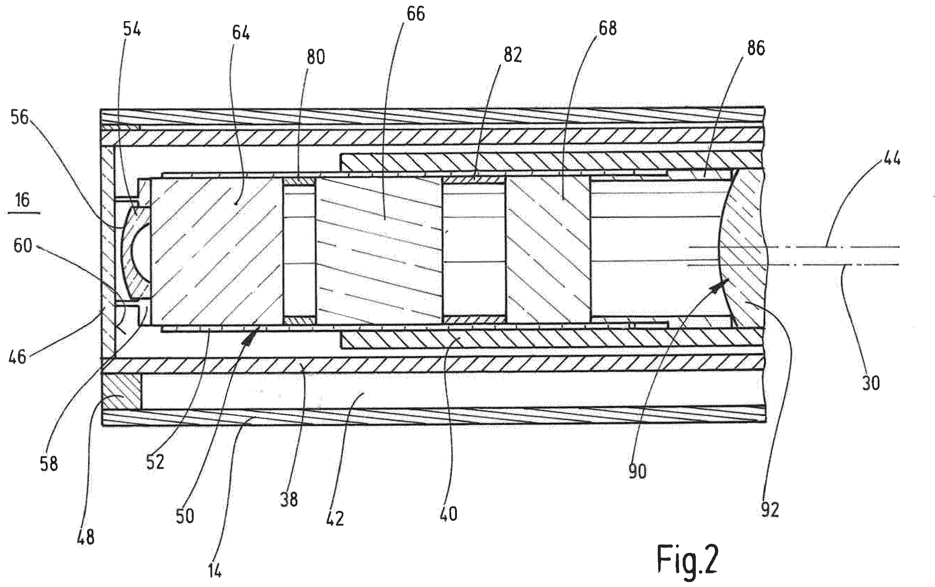

Das Instrument 10 kann mehrere Schäfte oder Rohre aufweisen, die zwischen dem distalen Ende 16 und dem proximalen Ende 18 angeordnet sind. In diesem Zusammenhang wird zu Veranschaulichungszwecken auf die vereinfachte Darstellung von Fig. 2 verwiesen. Insbesondere im Hinblick auf Wandstärken und sonstige Proportionen ist die Darstellung in Fig. 2 aus Veranschaulichungsgründen überzeichnet bzw. nicht maßstabsgerecht.

Fig. 2 zeigt einen distalen Abschnitt des Instrumentenschafts 14, der beim distalen Ende 16 angeordnet ist. Eine zentrale Längsachse des Instrumentenschafts 14 ist in Fig. 2 mit 30 bezeichnet. Im Instrumentenschaft 14 ist ein Zwischenschaft 38 aufgenommen. Im Zwischenschaft 38 ist ein Trägerschaft 40 aufgenommen. Der Trägerschaft 40, der Zwischenschaft 38 und der Instrumentenschaft 14 bilden insgesamt eine Gestaltung mit drei Rohren oder Schäften, die ineinander angeordnet sind. Ein Zwischenraum zwischen dem Zwischenschaft 38 und dem Instrumentenschaft 14 ist mit 42 bezeichnet. Beispielsweise sind der Zwischenschaft 38 und der Trägerschaft 40 konzentrisch zueinander ausgerichtet und mit einer gemeinsamen Längsachse 44 versehen. Beispielhaft ist der Instrumentenschaft 14 exzentrisch zum Zwischenschaft 38 bzw. zum Trägerschaft 40 ausgerichtet. Demgemäß ist im gezeigten Ausführungsbeispiel eine Längsachse 30 des Instrumentenschafts 14 von der (gemeinsamen) Längsachse 44 des Zwischenschafts 38 und des Trägerschafts 40 beabstandet. Es sind auch konzentrische Anordnungen der drei Schäfte 14, 38, 40 vorstellbar. Die Schäfte 14, 38, 40 sind regelmäßig zylindrisch gestaltet und mit kreisförmigen bzw. ringförmigen Querschnitten versehen. Jedoch sind auch abweichende, nicht kreisförmige Gestaltungen vorstellbar.

Beim distalen Ende 16 ist ein Abschlusselement 46 vorgesehen. Beispielhaft ist das distale Abschlusselement 46 am Zwischenschaft 38 aufgenommen. Ferner ist zwischen dem Instrumentenschaft 14 und dem Zwischenschaft 38 ein Verschluss 48 angedeutet. Es ist jedoch auch vorstellbar, das Abschlusselement 46 sowohl mit dem Zwischenschaft 38 als auch direkt mit dem Instrumentenschaft 14 zu koppeln. Üblicherweise ist der Instrumentenschaft 14 mit dem Zwischenschaft 38 beim distalen Ende 16 fest verbunden. Dies kann bspw. über eine stoffschlüssige Verbindung erfolgen, etwa mittels Kleben, Löten oder Schweißen. Es ist jedoch auch vorstellbar, einen Adapter zwischen dem Instrumentenschaft 14 und dem Zwischenschaft 38 vorzusehen, um den Verschluss zu bilden. Ferner können Lichtleiter, die im Zwischenraum 42 aufgenommen sind, einen Teil einer distalen Endfläche des Zwischenraums 42 füllen. Die Lichtleiter oder Lichtfasern können eingekittet sein. Wie vorstehend bereits ausgeführt, kann auch das Abschlusselement 46 die Kopplung zwischen dem Instrumentenschaft 14 und dem Zwischenschaft 38 bereitstellen.

Der Zwischenschaft 38 stellt eine Führung für den Trägerschaft 40 bereit. Im Zwischenschaft 38 ist eine Objektivbaugruppe 50 aufgenommen. Der Trägerschaft 40 dient als Träger für die Objektivbaugruppe 50. Beispielhaft ist die Objektivbaugruppe 50 in einer Hülse 52 aufgenommen, die mit dem Trägerschaft 40 verbunden ist. Es versteht sich, dass die Objektivbaugruppe 50 grundsätzlich auch direkt im Trägerschaft 40 aufgenommen sein kann. Die Verwendung der Hülse 52 kann genutzt werden, um die Objektivbaugruppe 50 zumindest teilweise vorzumontieren. Beispielsweise ist die Hülse 52 stoffschlüssig mit dem Trägerschaft 40 verbunden, etwa mittels Kleben, Löten oder Schweißen.

Die Objektivbaugruppe 50 umfasst verschiedene optische Bauelemente. Beispielsweise weist die Objektivbaugruppe 50 ein distalseitig angeordnetes Bauelement 54 auf, das etwa als asphärische Linse 56 gestaltet ist. Das Bauelement 54 bildet einen distalen Abschluss der Objektivbaugruppe 50. Fig. 2 zeigt ferner, dass sich die Objektivbaugruppe 50 über einen Abstandshalter 58 am distalen Abschlusselement 46, also an einem distalen Anschlag des Instruments 10 abstützt. Auf diese Weise kann insbesondere ein Abstand zwischen dem Abschlusselement 46 und dem Bauelement 54 hochgenau eingestellt werden. Das distale Abschlusselement 46 stellt eine Anlagefläche 60 bereit, an der sich der Abstandshalter 58 abstützt. Das Abschlusselement 46 kann auch halbmondartig oder in ähnlicher Weise gestaltet sein und muss demgemäß nicht zwingend ringförmig und geschlossen sein.

Die Objektivbaugruppe 50 umfasst neben dem Bauelement 54 bspw. weitere Bauelemente 64, 66, 68, die jeweils als Linsen, Prismen, Linsenpakete und/oder Prismenpakete gestaltet sind. Ferner können Abstandshalter 80, 82 vorgesehen sein, die etwa ringartig gestaltet sind. Insgesamt kann die Objektivbaugruppe 50, insbesondere bei Verwendung einer Hülse 52, eine kompakte Einheit bilden. Der Trägerschaft 40, der vorzugsweise fest mit der Objektivbaugruppe 50, insbesondere mit der Hülse 52, verbunden ist, ist in Richtung auf das distale Ende 16 vorgespannt. Auf diese Weise wird sichergestellt, dass das Bauelement 54 und ggf. weitere sich daran anschließende Bauelemente definiert am Abschlusselement 46 zur Anlage kommt/kommen.

In Fig. 2 ist ferner mit 86 ein Druckstück angedeutet, das zwischen der Objektivbaugruppe 50 und einer Stablinsenbaugruppe 90 angeordnet ist. Die Stablinsenbaugruppe 90 und die Objektivbaugruppe 50 sind der optischen Anordnung 34 zugeordnet, die auch eine Okularbaugruppe umfassen kann. Über das Druckstück 86 kann die Stablinsenbaugruppe 90 gegen die Objektivbaugruppe 50 gedrückt werden, etwa gegen ein proximales Bauelement 76 der Objektivbaugruppe 50. Die Stablinsenbaugruppe 90 weist eine Stablinse 92 auf, die auch als optisches Bauelement der Stablinsenbaugruppe 90 bezeichnet werden kann.

Bei der in Fig. 2 gezeigten beispielhaften Ausführungsform ist bspw. das Bauelement 64 mit der Hülse 52 verklebt oder auf andere Weise gefügt. Die Hülse 52 ist mit dem Trägerschaft 40 verklebt oder auf andere Weise gefügt. Das Bauelement 64 kontaktiert den Abstandshalter 58. Das Bauelement 54 ist mit dem Bauelement 64 und/oder dem Abstandshalter 58 gekoppelt. Eine Vorspannkraft kann über den Trägerschaft 40 auf die Hülse 52 und die über die Hülse 52 auf das Bauelement 64 aufgebracht werden. Somit kann das Bauelement 64 gegen den Abstandshalter 58 drücken und diesen in einen Kontakt mit der Anlagefläche 60 des Abschlusselements 46 bringen.

Eine weitere Vorspannung kann sich durch eine nach distal gerichtete Kraft ergeben, die auch auf die Stablinsenbaugruppe 90 einwirkt. Die Kraft kann über die Stablinse 92 auf das Druckstück 86 und über das Druckstück 86 auf die Elemente 68, 82, 66, 80, 64 der Objektivbaugruppe 50 einwirken, um diese in Richtung auf das Bauelement 64 vorzuspannen. Auf diese Weise wird einerseits der Trägerschaft 40 als auch die darin oder daran aufgenommene optische Anordnung 34 nach distal vorgespannt. Der Abstandshalter 58 definiert insgesamt eine axiale Position bzw. einen axialen Anschlag für die Komponenten der optischen Anordnung 34.

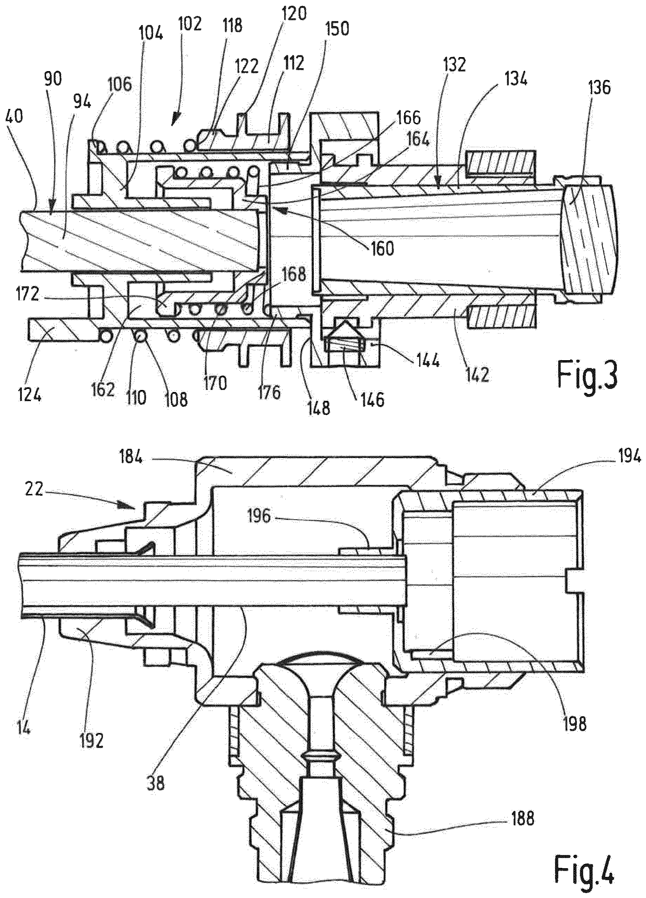

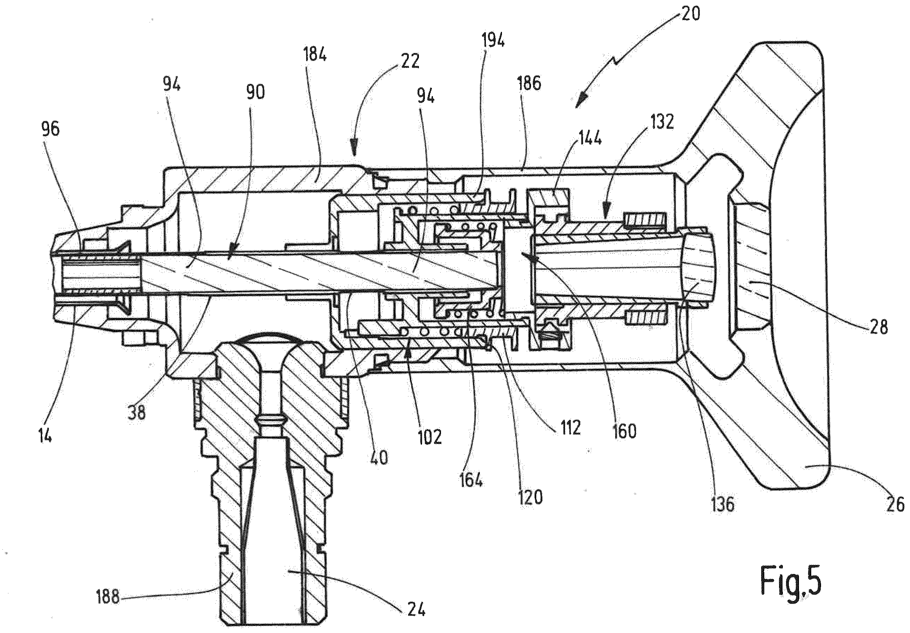

In Ergänzung zur Darstellung des distalen Abschnitts in Fig. 2 veranschaulichen die Figuren 3, 4 und 5 einen proximalen Abschnitt eines als Endoskop 12 gestalteten Instruments 10. Fig. 3 veranschaulicht Komponenten, die in einer in Fig. 4 dargestellten Gehäuseanordnung aufgenommen werden können. Fig. 5 zeigt einen montierten Zustand des proximalen Endes des Instruments 10. Im Vergleich zur überzeichneten Darstellung in Fig. 2 zeigen die Figuren 3, 4 und 5, dass die Schäfte 14, 38 und 40 jeweils nur geringe Wandstärken aufweisen.

Fig. 3 veranschaulicht ein proximales Ende des Trägerschafts 40, in dem die Stablinsenbaugruppe 90 aufgenommen ist. In Fig. 3 ist eine mit 94 bezeichnete proximale Stablinse teilweise dargestellt. Zwischen der in Fig. 2 mit 92 bezeichneten Stablinse und der in Fig. 3 mit 94 bezeichneten Stablinse können weitere Stablinsen, sonstige optische Bauelemente, Abstandshalter und Ähnliches im Trägerschaft 40 aufgenommen sein. Grundsätzlich sind diese Bauelemente, insbesondere auch die Stablinsen 92, 94 axial verschieblich im Trägerschaft 40 aufgenommen.

Mit dem Trägerschaft 40 ist eine Vorspanneinheit 102 gekoppelt. Die Vorspanneinheit 102 umfasst eine Aufnahmehülse 104, die an einem proximalen Ende des Trägerschafts 40 fest mit diesem verbunden ist. Bei der Aufnahmehülse 104 ist eine Mitnahmefläche 106 ausgebildet, die auch als distale Mitnahmefläche oder distaler Mitnahmebund bezeichnet werden kann. Ferner weist die Vorspanneinheit 102 ein Vorspannelement 108 auf, das etwa als Federelement 110 gestaltet ist. Das Vorspannelement 108 erstreckt sich zwischen einem Haltestück 112 und der Mitnahmefläche 106 der Aufnahmehülse 104. Das Haltestück 112 ist an einem proximalen Ende des Vorspannelements 108 angeordnet. Die Mitnahmefläche 106 ist an einem distalen Ende des Vorspannelements 108 angeordnet.

Am Haltestück 112 ist eine Druckfläche 118 für das Vorspannelement 108 ausgebildet. Ferner ist eine gehäuseseitige Anschlagfläche 120 ausgebildet, vgl. hierzu auch Fig. 5. Ferner umfasst das Haltestück 112 ein Gewinde 122, mit dem das Haltestück 112 mit einem Innengehäuse 194 (vgl. Fig. 4) koppelbar ist. Die Aufnahmehülse 104 umfasst beispielhaft eine Drehlagensicherung 124 in Form einer Nase am distalen Ende der Aufnahmehülse 104.

Das Vorspannelement 108 stützt sich an der Druckfläche 118 des Haltestücks 112 ab und drängt die Aufnahmehülse 104 nach distal. Den Figuren 3, 4 und 5 ist in Zusammenschau entnehmbar, dass das Haltestück 112 im gefügten Zustand über ein Innengehäuse 194 fest mit dem Kopfstück 20 oder Gehäuse 22 des Instruments 10 verbunden ist. Somit drängt das Vorspannelement 108 über die Aufnahmehülse 104 den Trägerschaft 40 in Richtung auf das distale Ende 16 des Instruments 10. Dies bewirkt schlussendlich einen Druck der Objektivbaugruppe 50 gegen den Abstandshalter 58, der sich am distalen Abschlusselement 46 abstützt.

Optische Bauelemente, insbesondere die Stablinsen 92, 94 der Stablinsenbaugruppe 90 und zumindest einige Bauelemente der Objektivbaugruppe 50, die axial verschieblich im Trägerschaft 40 aufgenommen sind, werden über eine weitere Vorspanneinheit 160 nach distal vorgespannt. Dies wird weiter unten detailliert erläutert.

Fig. 3 zeigt ferner, dass sich an die Stablinsenbaugruppe 90 eine Okularbaugruppe 132 anschließt. Die Okularbaugruppe 132 ist am proximalen Ende der Stablinsenbaugruppe 90 angeordnet. Die Objektivbaugruppe 50 (Fig. 2) ist beim distalen Ende der Stablinsenbaugruppe 90 angeordnet. Die Okularbaugruppe 132 umfasst beispielhaft einen Tubus 134, der zumindest ein optisches Bauelement 136 trägt, das etwa als Linse gestaltet ist. Im gefügten Zustand (vgl. Fig. 5) ist das Bauelement 136 der Okularbaugruppe 132 in der Nähe des Okularfensters 28 der Okularmuschel 26 angeordnet, um die optische Abbildung für einen Beobachter sichtbar zu machen. Die Okularbaugruppe 132 kann mehrere optische Bauelemente umfassen.

Fig. 3 zeigt, dass die Okularbaugruppe 132 über den Tubus 134 an einem hülsenartig gestalteten Träger 142 aufgenommen ist. Der Träger 142 ist mit einem Klemmstück 144 verbunden und über zumindest ein Klemmelement 146 am Klemmstück 144 festgelegt. Bei dem Klemmelement 146 handelt es sich beispielhaft um eine Madenschraube, die radial in eine Nut oder Ausnehmung am Umfang des Trägers 142 eingreift.

Das Klemmstück 144 weist eine Anschlagfläche 148 auf, die eine axiale Relativlage des Klemmstücks 144 in Bezug auf die Aufnahmehülse 104 definiert. Über ein Gewinde 150 kann das Klemmstück 144 kraftschlüssig mit der Aufnahmehülse 104 verbunden werden. Die Anschlagfläche 148 definiert eine Einschraubtiefe des Klemmstücks 144 an der Aufnahmehülse 104.

Die Vorspanneinheit 160 ist in einer Ausnehmung 162 der Aufnahmehülse 104 angeordnet. Die Ausnehmung 162 ist becherförmig oder als Ringnut gestaltet. Die Vorspanneinheit 160 umfasst ein Verbindungsstück 164, das etwa hülsenartig gestaltet ist. Das Verbindungsstück 164 ist mit einem proximalen Bauelement der Stablinsenbaugruppe 90, also etwa mit der Stablinse 94, gekoppelt. Die Vorspanneinheit 160 umfasst ein Vorspannelement 166. Das Vorspannelement 166 kann auch als inneres Vorspannelement bezeichnet werden. Im Gegensatz dazu kann das Vorspannelement 108 als äußeres Vorspannelement bezeichnet werden. Beispielhaft sind die Vorspannelemente 108, 166 konzentrisch zueinander ausgerichtet.

Das Vorspannelement 166 ist etwa als Feder 168, insbesondere als Schraubenfeder, gestaltet. Die Vorspannelemente 108, 166 sind beispielhaft als Druckfedern gestaltet. Das Vorspannelement 166 erstreckt sich zwischen dem Verbindungsstück 164 und dem Klemmstück 144. Die Vorspanneinheit 160 ist der Stablinsenbaugruppe 90 und der Okularbaugruppe 132 zwischengeschaltet.

Das Vorspannelement 166 stützt sich distal an einer Anschlagfläche 172 des Verbindungsstücks 164 ab. Das Verbindungsstück 164 weist einen Mitnahmebund 170 auf, der an einem proximalen Ende an die Stablinse 94 angreift und diese nach distal drängt. An seinem proximalen Ende stützt sich das Vorspannelement 166 an einer Anschlagfläche 176 ab, die am Klemmstück 144 ausgebildet ist. Die Vorspanneinheit 160 bewirkt eine axiale Vorspannung zwischen Bauelementen der Objektivbaugruppe 50, der Stablinsenbaugruppe 90 und der Okularbaugruppe 132. Auf diese Weise können die optischen Bauelemente (axial gesehen) nahezu spielfrei aufgenommen werden. Ferner können etwaige Längenänderungen, Stöße, Vibrationen und sonstige mechanische Belastungen zumindest teilweise kompensiert werden, ohne dass innere Spannungen entstehen, die Bauteilschäden bewirken können. Am distalen Ende des Trägerschafts 40 (Fig. 2) ist bspw. das Bauelement 64 (über die Hülse 52) fest mit dem Trägerschafts 40 verbunden. Auf diese Weise wird ein distaler Anschlag für die unter Vorspannung stehenden Bauelemente bereitgestellt.

Die Vorspanneinheit 102 drängt den Trägerschaft 40 in Richtung auf das distale Ende 16 des Instruments 10. Auf diese Weise wird eine hochgenaue distale axiale Ausrichtung und Positionierung der optischen Anordnung 34 ermöglicht, da durch den Kontakt mit dem Abstandshalter 58 eine distal axiale Lagereferenz bzw. ein Anschlag bereitgestellt wird.

Fig. 4 veranschaulicht anhand einer Schnittdarstellung die Gestaltung eines Gehäuses 22 eines Instruments 10, das zur Aufnahme der Fig. 3 gezeigten Anordnung ausgebildet ist. Das Gehäuse 22 umfasst bspw. einen distalen Gehäuseabschnitt 184, an den sich ein proximaler Gehäuseabschnitt 186 (nur in Fig. 5 gezeigt) anschließt. Fig. 5 zeigt ferner, dass der Gehäuseabschnitt 186 bspw. als Aufnahme für die Okularmuschel 26 sowie für das Okularfenster 28 dienen kann. Ferner weist das Gehäuse 22 einen Gehäuseabschnitt 188 auf, der den Lichtanschluss 24 beherbergt.

Am Gehäuseabschnitt 184 ist ein Führungsflansch 192 ausgebildet. Der Führungsflansch 192 definiert ein distales Ende des Gehäuses 22. Am Führungsflansch 192 ist der Instrumentenschaft 14 aufgenommen. Dies beinhaltet eine feste Kopplung des Instrumentenschafts 14 mit dem Führungsflansch 192. Dies kann bspw. eine kraftschlüssige, stoffschlüssige und/oder formschlüssige Kopplung umfassen.

Im Instrumentenschaft 14 ist der Zwischenschaft 38 aufgenommen. Der Zwischenschaft 38 und der Instrumentenschaft 14 sind parallel und versetzt zueinander (also nicht konzentrisch) angeordnet. Der Zwischenschaft 38 ragt am proximalen Ende des Instrumentenschafts 14 aus diesem heraus.

Am Gehäuse 22, insbesondere am Gehäuseabschnitt 184, ist ferner ein Innengehäuse 194 vorgesehen. Das Innengehäuse 194 ist fest mit dem Gehäuse 22 verbunden. Am Innengehäuse 194 ist ein Zwischenflansch 196 ausgebildet. Ein proximales Ende des Innengehäuses 194 ist mit dem Zwischenflansch 196 gekoppelt. Das Innengehäuse 194 dichtet über seinen Zwischenflansch 196 den Zwischenschaft 38 ab. Am Innengehäuse 194 ist ferner eine Drehlagensicherung 198 ausgebildet, die mit der Drehlagensicherung 124 der Aufnahmehülse 104 zusammenwirkt. Die Drehlagensicherung 124 ist beispielhaft als Nase gestaltet. Die Drehlagensicherung 198 ist bspw. als Nut oder Ausnehmung gestaltet, in die die Nase eingreifen kann. Auf diese Weise definiert das Innengehäuse 194 eine Drehlage der Aufnahmehülse 104 relativ zum Gehäuse 22.

Fig. 5 veranschaulicht die gefederte, zumindest teilweise "schwimmende" Lagerung der optischen Anordnung 34. Eine Gehäusereferenz für die Vorspanneinheit 102 ergibt sich durch die Anlage des Haltestücks 112 am gehäusefesten Innengehäuse 194. Somit stützt sich das Vorspannelement 108 mittelbar am Gehäuse 22 ab und drückt die Aufnahmehülse 104 und daran aufgenommene Komponenten in Richtung auf das distale Ende 16 des Instruments 10. Die Aufnahmehülse 104 ist axial verschieblich mit dem Haltestück 112 und somit mit dem Gehäuse 22 gekoppelt. An der Aufnahmehülse 104 ist das Klemmstück 144 aufgenommen, das eine Lagereferenz für die Okularbaugruppe 132 und somit auch für die Vorspanneinheit 160 definiert.

Das Vorspannelement 166 der Vorspanneinheit 160 stützt sich am Klemmstück 144 ab und drückt über das Verbindungsstück 164 die Stablinsenbaugruppe 90 nach distal. Dies kann eine axiale Relativbewegung zwischen der Stablinsenbaugruppe 90 (und ggf. weiteren optischen Bauelementen) sowie dem Trägerschaft 40 umfassen. Gemäß der in den Figuren 3, 4 und 5 veranschaulichten Ausgestaltung ist die Okularbaugruppe 132 über die Aufnahmehülse 104 fest (also ohne axiale Relativbewegung) mit dem Trägerschaft 40 gekoppelt. Fig. 5 zeigt ferner, dass der Trägerschaft 40 axial über das proximale Ende des Zwischenschafts 38 hinausragt.

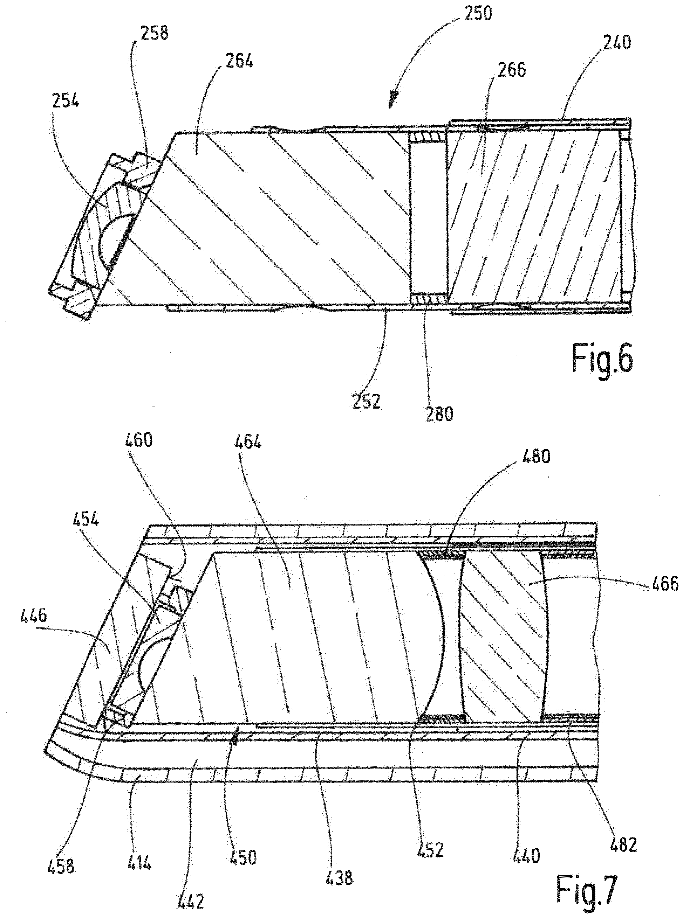

Die Figuren 6, 7, 8 und 9 veranschaulichen weitere beispielhafte Ausführungsformen von optischen Anordnungen für Beobachtungsinstrumente, bei denen in ähnlicher Weise ein (innerer) Trägerschaft nach distal vorgespannt ist, wobei ferner optische Bauelemente, die im Trägerschaft angeordnet sind, relativ zum Trägerschaft nach distal vorgespannt sind. Die in den Figuren 6, 7 und 8 beispielhaft veranschaulichten Komponenten zeigen jeweils Beobachtungsinstrumente mit seitlicher Blickrichtung, die einen Winkelversatz zur Längsachse des jeweiligen Instrumentenschafts aufweist.

Fig. 6 zeigt einen Schnitt durch einen distalen Abschnitt eines Trägerschafts 240. Aus Veranschaulichungsgründen wurde in Fig. 6 auf die entsprechende Darstellung des Instrumentenschafts und, sofern vorhanden, des Zwischenschafts, verzichtet.

Der Trägerschaft 240 trägt eine Objektivbaugruppe 250, die an einer Hülse 252 aufgenommen ist. Beispielhaft ist die Hülse 252 stoffschlüssig, kraftschlüssig, formschlüssig oder in sonstiger Weise mit dem Trägerschaft 240 gefügt. Die Objektivbaugruppe 250 umfasst verschiedene optische Bauelemente, etwa ein distal angeordnetes Bauelement 254. Ferner zeigt Fig. 6 einen Abstandshalter 258, über den die Objektivbaugruppe 250 in Richtung auf einen distalen Anschlag des Instruments vorgespannt werden kann. Dies kann durch eine axiale Vorspannung des Trägerschafts 240 bewirkt werden, der diese Vorspannung über die Hülse 252 auf die Objektivbaugruppe 250 überträgt. Somit kann die Objektivbaugruppe 250 gegen den Abstandshalter 258 gedrückt werden. Der Abstandshalter 258 kann eine gewünschte Soll-Lage für das Bauelement 254 gewährleisten.

Die Objektivbaugruppe 250 umfasst neben dem Bauelement 254 weitere Bauelemente oder Baugruppen 264, 266. Ferner ist beispielhaft zumindest ein Abstandshalter 280 vorgesehen. Zumindest einige der Bauelemente können relativ zum Trägerschaft 240 bzw. relativ zur Hülse 252 nach distal vorgespannt werden, so dass sich eine Anordnung analog zu den in den Figuren 2 bis 5 gezeigten Ausgestaltungen ergibt.

Fig. 7 zeigt einen Schnitt durch einen distalen Abschnitt eines Instrumentenschafts 414, in dem ein Trägerschaft 440 angeordnet ist, der eine Objektivbaugruppe 450 trägt. Die Gestaltung des Trägerschafts 440 sowie der Objektivbaugruppe 450 gemäß Fig. 7 entspricht grundsätzlich der bereits in Fig. 6 gezeigten Ausgestaltung.

Der Trägerschaft 440 ist in einem Zwischenschaft 438 aufgenommen, der im Instrumentenschaft 414 angeordnet ist. Zwischen dem Instrumentenschaft 414 und dem Zwischenschaft 438 ist ein Zwischenraum 442 definiert. Am distalen Ende des Instrumentenschafts 414 ist ein distales Abschlusselement 446 angeordnet, das bspw. im Zwischenschaft 438 aufgenommen ist. Das Abschlusselement 446 kann stoffschlüssig, formschlüssig, über Verbindungsteile oder in sonstiger Weise im Zwischenschaft 438 aufgenommen sein. Ferner ist beim distalen Ende eine feste Verbindung zwischen dem Instrumentenschaft 414 und dem Zwischenschaft 438 vorgesehen, die wiederum stoffschlüssig und/oder durch geeignete Verbindungselemente erzeugt werden kann.

Der Trägerschaft 440 trägt die Objektivbaugruppe 450, die wiederum zumindest teilweise in einer Hülse 452 aufgenommen ist. Die Hülse 452 ist mit dem Trägerschaft 440 verbunden. Der Trägerschaft 440 drückt die Hülse 452 und somit die Objektivbaugruppe 450 in Richtung auf das Abschlusselement 446. Die Objektivbaugruppe 450 umfasst ein Bauelement 454, das am distalen Ende der Objektivbaugruppe 450 angeordnet ist. Ferner umfasst die Objektivbaugruppe Bauelemente bzw. Baugruppen 464, 466 sowie Abstandshalter 480, 482.

Zumindest das Bauelement 454 oder das Bauelement 464 ist mit einem Abstandshalter 458 gekoppelt, um diesen nach distal in Richtung auf das Abschlusselement 446, insbesondere auf eine Anlagefläche 460 des Abschlusselements 446, zu drängen. Auf diese Weise kann ein gewünschter Abstand zwischen dem Abschlusselement 446 und dem Bauelement 454 herbeigeführt werden. Zumindest einige der Bauelemente der Objektivbaugruppe 450, insbesondere bei einem distalen Ende der Hülse 452, sind fest mit der Hülse 452 verbunden, etwa mittels Kleben, Löten oder einem ähnlichen geeigneten Fügeverfahren. Somit kann der Trägerschaft 440 über die Hülse 452 das entsprechende Bauelement beaufschlagen und den Abstandshalter 458 in einen Kontakt mit dem Abschlusselement 446 bringen. Die erforderliche Vorspannkraft kann über eine äußere Vorspanneinheit aufgebracht werden (vgl. die Vorspanneinheit 102 in Fig. 3).

Zumindest einige der Bauelemente der Objektivbaugruppe 450, insbesondere beim proximalen Ende der Hülse 452, können grundsätzlich axial verschieblich ausgeführt sein. Auf diese Weise kann eine Vorspannkraft, die etwa über eine innere Vorspanneinheit (vgl. die Vorspanneinheit 160 in Fig. 3) aufgebracht werden kann, in Richtung auf das distale Ende der Objektivbaugruppe 450 übertragen werden. Dies kann die gewünschte axiale Ausrichtung der Elemente der optischen Anordnung im Instrumentenschaft 414 ermöglichen. Insbesondere kann die Vorspannkraft über die Elemente einer Stablinsenbaugruppe übertragen werden, die in den Figuren 6 und 7 nicht explizit dargestellt ist.

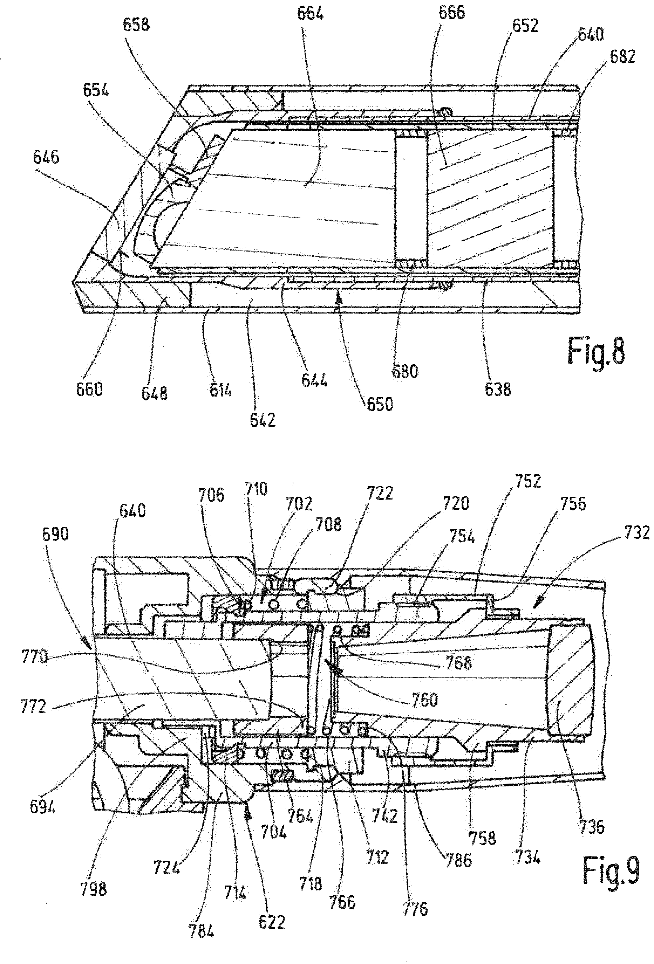

Fig. 8 veranschaulicht eine weitere Schnittansicht eines distalen Abschnitts eines Instrumentenschafts 614. Im Instrumentenschaft 614 ist ein Zwischenschaft 638 aufgenommen. Im Zwischenschaft 638 ist ein Trägerschaft 640 aufgenommen. Zwischen dem Instrumentenschaft 614 und dem Zwischenschaft 638 ist ein Zwischenraum 642 definiert. Der Zwischenschaft 638 weist an seinem distalen Ende ein Endstück 644 auf. Am Endstück 644 ist ein distales Abschlusselement 646 aufgenommen. In Fig. 8 sind alle drei Schäfte 614, 638, 640 konzentrisch zueinander ausgerichtet.

Zwischen dem Instrumentenschaft 614 und dem Endstück 644 des Zwischenschafts 638 ist ein Verschlussring 648 angeordnet, der beispielhaft das Endstück 644 und das darin aufgenommene Abschlusselement 646 umgibt. Auf diese Weise ist das distale Ende des Instrumentenschafts 614 hermetisch abgedichtet, dies umfasst eine feste Verbindung zwischen dem Zwischenschaft 638 und dem Instrumentenschaft 614. Der Trägerschaft 640 trägt eine Objektivbaugruppe 650. Die Objektivbaugruppe 650 umfasst eine Hülse 652, die mit dem Trägerschaft 640 gekoppelt ist. Grundsätzlich kann der Trägerschaft 640 die Hülse 652 auch integral ausbilden.

Die Objektivbaugruppe 650 umfasst ein Bauelement 654, das am distalen Ende der Objektivbaugruppe 650 ausgebildet ist. Ein Abstand zwischen dem Bauelement 654 und dem Abschlusselement 646 wird durch einen Abstandshalter 658 definiert, der zwischen der Objektivbaugruppe 650 und dem Abschlusselement 646 angeordnet ist und eine Anlagefläche 660 des Abschlusselements 646 kontaktiert. Ferner umfasst die Objektivbaugruppe 650 Bauelemente bzw. Baugruppen 664, 666. Daneben zeigt Fig. 8 bspw. ringartig gestaltete Abstandshalter 680, 682.

Fig. 9 veranschaulicht einen Schnitt durch einen proximalen Abschnitt eines Gehäuses 622 eines Beobachtungsinstruments. Der in Fig. 8 gezeigte distale Abschnitt und der in Fig. 9 gezeigte proximale Abschnitt können ein und derselben Ausführungsform zugeordnet sein, wobei die Darstellung nicht maßstabsgerecht ist. Die Darstellung in Fig. 8 ist gegenüber der Darstellung in Fig. 9 vergrößert.

Das in Fig. 9 in Teilschnittdarstellung gezeigte Gehäuse 622 ist grundsätzlich dem in den Figuren 4 und 5 gezeigten Gehäuse 22 ähnlich gestaltet. Der in Fig. 8 bereits teilweise gezeigte Trägerschaft 640 erstreckt sich zumindest teilweise in das in Fig. 9 gezeigte Gehäuse 622. Am oder im Trägerschaft 640 ist neben der in Fig. 8 gezeigten Objektivbaugruppe 650 noch eine Stablinsenbaugruppe 90 angeordnet, die bspw. eine Stablinse 694 umfasst.

Das Gehäuse 622 umfasst Gehäuseabschnitte 684, 686, von denen der Gehäuseabschnitt 684 distalseitig und der Gehäuseabschnitt 686 proximalseitig angeordnet sind.

Ferner veranschaulicht Fig. 9 eine mit 732 bezeichnete Okularbaugruppe, die der in Fig. 3 gezeigten Okularbaugruppe 132 zumindest ähnlich gestaltet ist. Die Okularbaugruppe 732 umfasst einen Tubus 734, der zumindest ein Bauelement 736 trägt. In Zusammenschau zeigen die Figuren 8 und 9, dass die Stablinsenbaugruppe 690 zwischen der Objektivbaugruppe 650 und der Okularbaugruppe 732 angeordnet ist.

Ähnlich wie bereits anhand der Figuren 3, 4 und 5 veranschaulicht, weist auch die Gestaltung gemäß den Figuren 8 und 9 eine erste Vorspanneinheit 702 und eine zweite Vorspanneinheit 760 auf. Die erste Vorspanneinheit 702 kann auch als äußere Vorspanneinheit bezeichnet werden. Die zweite Vorspanneinheit 760 kann auch als innere Vorspanneinheit bezeichnet werden. Die Vorspanneinheit 702 dient dazu, eine nach distal gerichtete Vorspannkraft auf den Trägerschaft 640 zu erzeugen. Die Vorspanneinheit 760 dient dazu, eine axiale Vorspannkraft zur axialen Positionierung von Elementen der optischen Anordnung des Beobachtungsinstruments zu bewirken. Somit kann die Vorspanneinheit 760 etwa Komponenten der Stablinsenbaugruppe 690 und ggf. auch der Objektivbaugruppe 650 nach distal vorspannen.

Die Vorspanneinheit 702 weist eine Aufnahmehülse 704 auf, die fest mit dem Trägerschaft 640 gekoppelt ist. Bei der Aufnahmehülse 704 ist ein Stützring 714 vorgesehen, an dem eine Mitnahmefläche 706 ausgebildet ist, an die ein distales Ende eines Vorspannelements 708 angreift, das bspw. als Schraubenfeder 710 gestaltet ist. An seinem proximalen Ende stützt sich das Vorspannelement 708 an einer Druckfläche 718 eines Haltestücks 712 ab. Das Haltestück 712 umfasst ferner eine Anschlagfläche 720 zur Anlage am Gehäuse 622. Beispielhaft kann das Haltestück 712 über ein Gewinde 722 mit dem Gehäuseabschnitt 784 des Gehäuses 622 gekoppelt werden. Auf diese Weise definiert das Haltestück 712 eine axiale Referenz bzw. einen axialen Anschlag für das Vorspannelement 708 der Vorspanneinheit 702. Die Vorspanneinheit 702 drängt den Trägerschaft 640 in Richtung auf das distale Ende des Instrumentenschafts 614 und folglich über den Abstandshalter 658 in Richtung auf das distale Abschlusselement 646.

Die Aufnahmehülse 704 weist ferner eine Drehlagensicherung 724 in Form einer Ausnehmung auf, die mit einer Drehlagensicherung 798 in Form einer Nase am Gehäuse 622 zusammenwirkt.

Die Okularbaugruppe 732 ist über dem Tubus 734 an einem Träger 742 aufgenommen. Gemäß dem in Fig. 9 gezeigten Ausführungsbeispiel ist der Träger 742 durch einen Abschnitt der Aufnahmehülse 704 gebildet. Mit anderen Worten ist der Tubus 734 an der Aufnahmehülse 704 aufgenommen. Zur Lagesicherung dient ein Überwurfdeckel 752, der über ein Gewinde 754 mit der Aufnahmehülse 704 gekoppelt ist. Der Überwurfdeckel 752 weist eine Druckfläche 756 auf, die an einem Mitnahmebund 758 des Tubus 734 angreift, um diesen am Träger 742 festzulegen.

Die Vorspanneinheit 760 umfasst ein Vorspannelement 766, das bspw. als Schraubenfeder 768 gestaltet ist. Das Vorspannelement 766 erstreckt sich zwischen einem Verbindungsstück 764 und dem Tubus 734. Am Verbindungsstück 764 ist ein Mitnahmebund 770 ausgebildet. Das Verbindungsstück 764 ist mit der Stablinse 694 gekoppelt. Über den Mitnahmebund 770 kann das Verbindungsstück 764 eine nach distal gerichtete Vorspannkraft auf die Stablinsenbaugruppe 690 übertragen. Ferner weist das Verbindungsstück 764 eine Anschlagfläche 772 für ein distales Ende des Vorspannelements 766 auf. Am Tubus 734 ist eine Anschlagfläche 776 für ein proximales Ende des Vorspannelements 766 ausgebildet. Somit drängt das Vorspannelement 766 die Stablinsenbaugruppe 90 in Richtung auf das distale Ende, also in Richtung auf die Objektivbaugruppe 650. Ferner drängt das Vorspannelement 766 die Okularbaugruppe 732 in Richtung auf einen proximalen Anschlag, der beispielhaft durch die Druckfläche 756 und den Mitnahmebund 758 gebildet ist.

Ein Unterschied zwischen den anhand der Figuren 3 bis 5 sowie anhand der Figuren 8 und 9 veranschaulichten Ausgestaltungen besteht darin, dass die Okularbaugruppe 732 kraftschlüssig durch das Vorspannelement 708 gegen einen proximalen Anschlag gedrückt wird. Hingegen ist die Okularbaugruppe 132 (vgl. Fig. 3 und Fig. 5) über das Klemmstück 144 fest (sowohl proximal als auch distal lagefest) mit der Aufnahmehülse 104 gekoppelt.

Gemäß der in Fig. 9 gezeigten Ausgestaltung kann die Okularbaugruppe 732 grundsätzlich nach distal relativ zur Aufnahmehülse 704 bewegt werden, obgleich hierzu eine durch die Vorspanneinheit 760 aufgebracht Kraft überwunden werden muss.

Sowohl gemäß der in den Figuren 3 und 5 als auch gemäß der in Fig. 9 veranschaulichten Ausgestaltung sind die Aufnahmehülsen 104, 704 axial verschieblich am Gehäuse 22, 622 aufgenommen.

## Patentansprüche

1. Beobachtungsinstrument, insbesondere Endoskop, mit einem Instrumentenschaft (14, 414, 614) mit einem distalen Ende (16) und einem proximalen Ende (18), und mit einer optischen Anordnung (34), die sich zumindest abschnittsweise durch den Instrumentenschaft (14, 414, 614) erstreckt, wobei die optische Anordnung (34) zumindest ein optisches Bauelement (54, 92, 94, 254, 454, 654, 694) aufweist, das in einem Trägerschaft (40, 240, 440, 640) aufgenommen ist, wobei der Trägerschaft (40, 240, 440, 640) im Instrumentenschaft (14, 414, 614) zumindest abschnittsweise axial verschieblich aufgenommen und nach distal vorgespannt ist, und wobei der Trägerschaft (40, 240, 440, 640) durch ein proximal angeordnetes Vorspannelement (108, 708), das auf den Trägerschaft (40, 240, 440, 640) einwirkt, nach distal vorgespannt ist, **dadurch gekennzeichnet, dass** die optische Anordnung (34) im Trägerschaft (40, 240, 440, 640) durch ein weiteres Vorspannelement (166, 766) zumindest abschnittsweise relativ zum Trägerschaft (40, 240, 440, 640) nach distal vorgespannt ist.

2. Beobachtungsinstrument nach Anspruch 1, **dadurch gekennzeichnet, dass** der Trägerschaft (40, 240, 440, 640) im Instrumentenschaft (14, 414, 614) axial gegen ein distales Abschlusselement (46, 446, 646) vorgespannt ist, das lagefest mit dem Instrumentenschaft (14, 414, 614) gekoppelt ist.

3. Beobachtungsinstrument nach Anspruch 2, **gekennzeichnet durch** einen Abstandshalter (58, 258, 458, 658), der zwischen dem Trägerschaft (40, 240, 440, 640) und dem distalen Abschlusselement (46, 446, 646) angeordnet ist.

4. Beobachtungsinstrument nach Anspruch 3, **dadurch gekennzeichnet, dass** der Abstandshalter (58, 258, 458, 658) einen Abstand zwischen einer Objektivbaugruppe (50, 250, 450, 650) der optischen Anordnung (34) und dem distalen Abschlusselement (46, 446, 646) definiert.

5. Beobachtungsinstrument nach einem der vorhergehenden Ansprüche, **dadurch gekennzeichnet, dass** sich der Trägerschaft (40, 240, 440, 640) ausgehend vom distalen Ende (16) des Instrumentenschafts (14, 414, 614) hin zum proximalen Ende (18) des Instrumentenschafts (14, 414, 614) erstreckt.

6. Beobachtungsinstrument nach Anspruch 5, **dadurch gekennzeichnet, dass** sich der Trägerschaft (40, 240, 440, 640) in ein Gehäuse (22, 622) eines Kopfstücks (20) des Beobachtungsinstruments erstreckt.

7. Beobachtungsinstrument nach einem der vorhergehenden Ansprüche, **dadurch gekennzeichnet, dass** das Vorspannelement, das den Trägerschaft (40, 240, 440, 640) nach distal vorspannt, eine Druckfeder (110, 710) ist.

8. Beobachtungsinstrument nach einem der vorhergehenden Ansprüche, **dadurch gekennzeichnet, dass** das Vorspannelement (108, 708) zwischen einer proximalen Druckfläche (118) und einer distalen Mitnahmefläche (106) angeordnet ist, die mit dem Trägerschaft (40, 240, 440, 640) gekoppelt ist.

9. Beobachtungsinstrument nach einem der vorhergehenden Ansprüche, **dadurch gekennzeichnet, dass** das Vorspannelement (108, 708) einen Beobachtungsstrahlengang (36) des Beobachtungsinstruments umgibt.

10. Beobachtungsinstrument nach einem der vorhergehenden Ansprüche, **dadurch gekennzeichnet, dass** die optische Anordnung (34) zumindest teilweise im Trägerschaft (40, 240, 440, 640) axial verschieblich aufgenommen ist und durch das weitere Vorspannelement (166, 766), das als inneres Vorspannelement gestaltet ist, nach distal vorgespannt wird.

11. Beobachtungsinstrument nach Anspruch 10, **dadurch gekennzeichnet, dass** das innere Vorspannelement (166, 766) mit zumindest einem optischen Bauelement (94, 694), insbesondere einer Stablinse, gekoppelt ist, das im Trägerschaft (40, 240, 440, 640) angeordnet ist und das zumindest eine optische Bauelement (94, 694) relativ zum Trägerschaft (40, 240, 440, 640) nach distal vorspannt.

12. Beobachtungsinstrument nach Anspruch 10 oder 11, **dadurch gekennzeichnet, dass** die optische Anordnung (34) ferner eine Okularbaugruppe (132, 732) umfasst, die proximalseitig des Trägerschafts (40, 240, 440, 640) angeordnet ist, und dass sich das innere Vorspannelement (166, 766) an seinem proximalen Ende an der Okularbaugruppe (132, 732) abstützt.

13. Beobachtungsinstrument nach einem der vorhergehenden Ansprüche, **dadurch gekennzeichnet, dass** zwischen dem Instrumentenschaft (14, 414, 614) und dem Trägerschaft (40, 240, 440, 640) ein Zwischenschaft (38, 438, 638) angeordnet ist, der den Trägerschaft (40, 240, 440, 640) umgibt.

14. Beobachtungsinstrument nach Anspruch 13, **dadurch gekennzeichnet, dass** der Zwischenschaft (38, 438, 638) beim distalen Ende fest mit dem Instrumentenschaft (14, 414, 614) verbunden ist, und dass vorzugsweise ein distales Abschlusselement (46, 446, 646) im Zwischenschaft (38, 438, 638) aufgenommen ist.

15. Beobachtungsinstrument nach Anspruch 13 oder 14, **dadurch gekennzeichnet, dass** der Zwischenschaft (38, 438, 638) den Trägerschaft (40, 240, 440, 640) und die darin aufgenommene optische Anordnung (34) nach außen abschließt.

## Claims

1. An observation instrument, in particular an endoscope, comprising an instrument shaft (14, 414, 614) having a distal end (16) and a proximal end (18), and comprising an optical arrangement (34) at least sectionally extending through the instrument shaft (14, 414, 614), wherein the optical arrangement (34) comprises at least one optical component (54, 92, 94, 254, 454, 654, 654, 694) that is arranged in a support shaft (40, 240, 440, 640), wherein the support shaft (40, 240, 440, 640) is at least sectionally axially slidably supported and distally preloaded in the instrument shaft (14, 414, 614), and wherein the support shaft (40, 240, 440, 640) is preloaded distally by a biasing element (108, 708) that is proximally arranged and that acts on the support shaft (40, 240, 440, 640), **characterized in that** the optical arrangement (34) in the support shaft (40, 240, 440, 640) is at least sectionally preloaded distally, relative to the support shaft (40, 240, 440, 640), by a further biasing element (166, 766).

2. The observation instrument according to claim 1, **characterized in that** the support shaft (40, 240, 440, 640) is in the instrument shaft (14, 414, 614) axially preloaded against a distal end element (46, 446, 646) that is fixedly coupled to the instrument shaft (14, 414, 614).

3. The observation instrument according to claim 2, **characterized by** a spacer (58, 258, 458, 658) arranged between the support shaft (40, 240, 440, 640) and the distal end element (46, 446, 646).

4. The observation instrument according to claim 3, **characterized in that** the spacer (58, 258, 458, 658) defines a distance between an objective lens assembly (50, 250, 450, 650) of the optical arrangement (34) and the distal end element (46, 446, 646).

5. The observation instrument according to any one of the preceding claims, **characterized in that** the support shaft (40, 240, 440, 640) extends from the distal end (16) of the instrument shaft (14, 414, 614) to the proximal end (18) of the instrument shaft (14, 414, 614).

6. The observation instrument according to claim 5, **characterized in that** the support shaft (40, 240, 440, 640) extends into a housing (22, 622) of a head piece (20) of the observation instrument.

7. The observation instrument according to any one of the preceding claims, **characterized in that** the biasing element, that preloads the support shaft (40, 240, 440, 640) distally, is a compression spring (110, 710).

8. The observation instrument according to any one of the preceding claims, **characterized in that** the biasing element (108, 708) is disposed between a proximal pressure surface (118) and a distal driving surface (106) that is coupled to the support shaft (40, 240, 440, 640).

9. The observation instrument according to any one of the preceding claims, **characterized in that** the biasing element (108, 708) surrounds an observation beam path (36) of the observation instrument.

10. The observation instrument according to any one of the preceding claims, **characterized in that** the optical arrangement (34) is at least partially axially slidably accommodated in the support shaft (40, 240, 440, 640) and preloaded distally by the further biasing element (166, 766), which is arranged as an inner biasing element (166, 766).

11. The observation instrument according to claim 10, **characterized in that** the inner biasing element (166, 766) is coupled to at least one optical component (94, 694), in particular a rod lens, which is arranged in the support shaft (40, 240, 440, 640), and wherein the inner biasing element (166, 766) preloads the at least one optical component (94, 694) distally relative to the support shaft (40, 240, 440, 640).

12. The observation instrument according to claim 10 or 11, **characterized in that** the optical arrangement (34) further comprises an ocular assembly (132, 732) arranged proximal of the support shaft (40, 240, 440, 640), and **in that** the inner biasing element (166, 766) is supported at its proximal end at the ocular assembly (132, 732).

13. Observation instrument according to any one of the preceding claims, **characterized in that** between the instrument shaft (14, 414, 614) and the support shaft (40, 240, 440, 640) an intermediate shaft (38, 438, 638) is arranged that surrounds the support shaft (40, 240, 440, 640).

14. The observation instrument according to claim 13, **characterized in that** the intermediate shaft (38, 438, 638) is at the distal end fixedly attached to the instrument shaft (14, 414, 614), and **in that** preferably a distal end element (46, 446, 646) is arranged in the intermediate shaft (38, 438, 638).

15. The observation instrument according to claim 13 or 14, **characterized in that** the intermediate shaft (38, 438, 638) closes the support shaft (40, 240, 440, 640) and the optical arrangement (34) arranged therein to the outside.

## Revendications

1. Instrument d'observation, en particulier endoscope, pourvu d'une tige d'instrument (14, 414, 614) possédant une extrémité distale (16) et une extrémité proximale (18), et pourvu d'un dispositif optique (34), lequel s'étend au moins partiellement à travers la tige d'instrument (14, 414, 614), dans lequel le dispositif optique (34) comporte au moins un composant optique (54, 92, 94, 254, 454, 654, 694), lequel est logé dans une tige de support (40, 240, 440, 640), dans lequel la tige de support (40, 240, 440, 640) est logée au moins partiellement dans la tige d'instrument (14, 414, 614) de manière à pouvoir coulisser axialement et est précontrainte vers l'extrémité distale, et dans lequel la tige de support (40, 240, 440, 640) est précontrainte vers l'extrémité distale par le biais d'un élément de précontrainte (108, 708) agencé à l'extrémité proximale et opérant sur la tige de support (40, 240, 440, 640), **caractérisé en ce que** le dispositif optique (34) dans la tige de support (40, 240, 440, 640) est au moins partiellement précontraint vers l'extrémité distale par rapport à la tige de support (40, 240, 440, 640) par le biais d'un élément de précontrainte supplémentaire (166, 766).

2. Instrument d'observation selon la revendication 1, **caractérisé en ce que** la tige de support (40, 240, 440, 640) est axialement précontrainte dans la tige d'instrument (14, 414, 614) contre un élément terminal distal (46, 446, 646), lequel est couplé en position fixe avec la tige d'instrument (14, 414, 614).

3. Instrument d'observation selon la revendication 2, **caractérisé par** un écarteur (58, 258, 458, 658), lequel est agencé entre la tige de support (40, 240, 440, 640) et l'élément terminal distal (46, 446, 646).

4. Instrument d'observation selon la revendication 3, **caractérisé en ce que** l'écarteur (58, 258, 458, 658) définit un écart entre un composant d'objectif (50, 250, 450, 650) du dispositif optique (34) et l'élément terminal distal (46, 446, 646).

5. Instrument d'observation selon l'une des revendications précédentes, **caractérisé en ce que** la tige de support (40, 240, 440, 640) s'étend de l'extrémité proximale (18) de la tige d'instrument (14, 414, 614) jusqu'à saillir de l'extrémité distale (16) de la tige d'instrument (14, 414, 614).

6. Instrument d'observation selon la revendication 5, **caractérisé en ce que** la tige de support (40, 240, 440, 640) s'étend dans un boîtier (22, 622) d'une pièce de tête (20) de l'instrument d'observation.

7. Instrument d'observation selon l'une des revendications précédentes, **caractérisé en ce que** l'élément de précontrainte précontraignant la tige de support (40, 240, 440, 640) vers l'extrémité distale est un ressort de compression (110, 710).

8. Instrument d'observation selon l'une des revendications précédentes, **caractérisé en ce que** l'élément de précontrainte (108, 708) est agencé entre une surface de pression proximale (118) et une surface de logement distale (106), laquelle est couplée avec la tige de support (40, 240, 440, 640).

9. Instrument d'observation selon l'une des revendications précédentes, **caractérisé en ce que** l'élément de précontrainte (108, 708) entoure un chemin optique d'observation (36) de l'instrument d'observation.

10. Instrument d'observation selon l'une des revendications précédentes, **caractérisé en ce que** le dispositif optique (34) est logé au moins partiellement dans la tige de support (40, 240, 440, 640) de manière à pouvoir coulisser axialement et est précontraint vers l'extrémité distale par le biais de l'élément de précontrainte supplémentaire (166, 766), lequel est configuré comme élément interne de précontrainte.

11. Instrument d'observation selon la revendication 10, **caractérisé en ce que** l'élément interne de précontrainte (166, 766) est couplé avec au moins un composant optique (94, 694), en particulier une lentille à tige, lequel est agencé dans la tige de support (40, 240, 440, 640) et précontraint vers l'extrémité distale l'au moins un composant optique (94, 694) par rapport à la tige de support (40, 240, 440, 640).

12. Instrument d'observation selon la revendication 10 ou 11, **caractérisé en ce que** le dispositif optique (34) comprend en outre un composant d'oculaire (132, 732), lequel est agencé côté proximal de la tige de support (40, 240, 440, 640), et **en ce que** l'élément interne de précontrainte (166, 766) s'appuie à son extrémité proximale sur le composant d'oculaire (132, 732).

13. Instrument d'observation selon l'une des revendications précédentes, **caractérisé en ce qu'**une tige intermédiaire (38, 438, 638) est agencée entre la tige d'instrument (14, 414, 614) et la tige de support (40, 240, 440, 640) et entoure la tige de support (40, 240, 440, 640).

14. Instrument d'observation selon la revendication 13, **caractérisé en ce que** la tige intermédiaire (38, 438, 638) est raccordée fixement à la tige d'instrument (14, 414, 614) à l'extrémité distale et **en ce que** de préférence un élément terminal distal (46, 446, 646) est logé dans la tige intermédiaire (38, 438, 638).

15. Instrument d'observation selon la revendication 13 ou 14, **caractérisé en ce que** la tige intermédiaire (38, 438, 638) ferme vers l'extérieur la tige de support (40, 240, 440, 640) et le système optique (34) logé dans celle-ci.
